(19) Europäisches Patentamt / European Patent Office / Office européen des brevets

(11) **EP 3 408 674 B1**

(12) **FASCICULE DE BREVET EUROPEEN**

(45) Date de publication et mention de la délivrance du brevet:
**08.01.2020 Bulletin 2020/02**

(21) Numéro de dépôt: **17707596.7**

(22) Date de dépôt: **25.01.2017**

(51) Int Cl.:
*G01N 33/68* (2006.01)  *G01N 33/86* (2006.01)

(86) Numéro de dépôt international:
**PCT/FR2017/050158**

(87) Numéro de publication internationale:
**WO 2017/129895 (03.08.2017 Gazette 2017/31)**

(54) **PROCÉDÉ DE DIAGNOSTIC DE LA CIVD**

VERFAHREN ZUR DIAGNOSE VON DIC

METHOD FOR DIAGNOSING DIC

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **27.01.2016 FR 1650667**
**29.01.2016 FR 1650765**
**01.12.2016 FR 1661823**

(43) Date de publication de la demande:
**05.12.2018 Bulletin 2018/49**

(73) Titulaires:
• **Hôpitaux Universitaires de Strasbourg (HUS)**
**67000 Strasbourg (FR)**
• **Diagnostica Stago**
**92600 Asnières-sur-Seine (FR)**

(72) Inventeurs:
• **BOISRAMÉ-HELMS, Julie**
**67000 Strasbourg (FR)**
• **TOTI, Florence**
**67400 Illkirch (FR)**
• **STIEL, Laure**
**67000 Strasbourg (FR)**
• **MAUVIEUX, Laurent**
**67450 Mundolsheim (FR)**
• **MEZIANI, Ferhat**
**67205 Oberhausbergen (FR)**

(74) Mandataire: **Novagraaf Technologies**
**Bâtiment O2**
**2, rue Sarah Bernhardt**
**CS90017**
**92665 Asnières-sur-Seine Cedex (FR)**

(56) Documents cités:
**EP-A2- 2 587 262    WO-A1-2014/184478**

• **SONG ET AL: "Neutrophil CD64 expression is associated with severity and prognosis of disseminated intravascular coagulation", THROMBOSIS RESEARCH, TARRYTOWN, NY, US, vol. 121, no. 4, 1 janvier 2008 (2008-01-01), pages 499-507, XP022449846, ISSN: 0049-3848, DOI: 10.1016/J.THROMRES.2007.05.013**
• **S. H. PARK ET AL: "Sepsis affects most routine and cell population data (CPD) obtained using the Sysmex XN-2000 blood cell analyzer: neutrophil-related CPD NE-SFL and NE-WY provide useful information for detecting sepsis", INTERNATIONAL JOURNAL OF LABORATORY HEMATOLOGY, vol. 37, no. 2, 28 avril 2015 (2015-04-28) , pages 190-198, XP055359090, GB; US ISSN: 1751-5521, DOI: 10.1111/ijlh.12261**
• **KOICHIRO SUEYOSHI ET AL: "Fluorescence imaging of ATP in neutrophils from patients with sepsis using organelle-localizable fluorescent chemosensors", ANNALS OF INTENSIVE CARE, vol. 6, no. 1, 15 décembre 2016 (2016-12-15), XP055356673, DOI: 10.1186/s13613-016-0175-z**

**Description**

**Domaine technique**

La présente invention se rapporte à un procédé de prédiction et/ou de détection de la coagulation intravasculaire disséminée (CIVD) dans un échantillon biologique.

La présente invention se rapporte également à un procédé de suivi de l'évolution d'une coagulation intravasculaire disséminée (CIVD) à partir d'un échantillon biologique.

La présente invention trouve une application notamment dans le domaine médical et vétérinaire.

Dans la description ci-dessous, les références entre crochets ([]) renvoient à la liste des références présentées à la fin du texte.

**État de la technique**

Le choc septique est un syndrome caractérisé par une réaction inflammatoire de l'hôte en réponse à un agent pathogène responsable d'une hypotension résistante à l'expansion volémique et nécessitant l'introduction de vasopresseurs (noradrénaline) et s'accompagnant de défaillances viscérales.

La coagulation est activée de manière constante au cours du choc septique et participe à la défense de l'hôte. Toutefois, cette activation peut être « dérégulée » avec une thrombopénie, une stimulation leucocytaire et endothéliale ainsi qu'une consommation des facteurs de la coagulation et de ses inhibiteurs, se traduisant par des microthromboses et responsable d'une coagulation intravasculaire disséminée (CIVD). L'association entre CIVD, gravité et défaillance multiviscérale est connue de longue date, rationnel de l'utilisation de traitements anticoagulants au cours du choc septique en plus des traitements étiologiques et de défaillances d'organes. Toutefois, les études cliniques portant sur des traitements ayant un impact sur la coagulation - qui n'ont jamais ciblé spécifiquement les patients présentant une CIVD - se sont révélées décevantes car ne permettant pas d'améliorer le pronostic des patients.

À l'échelle de la France, 80000 cas de sepsis sont observés chaque année, défini comme une réponse inflammatoire systémique que l'organisme développe en réponse à l'invasion par un micro-organisme pathogène, et qui se traduit par une hypoperfusion des organes et une dette en oxygène, pouvant évoluer vers un tableau de défaillance multiviscérale. L'hypotension artérielle y est persistante malgré un remplissage vasculaire adapté et nécessite le recours à des vasopresseurs (noradrénaline, adrénaline). Il s'agit donc d'une maladie grave et assez fréquente.

Cet état est lié à une activation cellulaire intense via l'induction de cytokines pro-inflammatoires et des modifications phénotypiques de l'endothélium vasculaire, qui prend alors un caractère pro-adhésif, pro-inflammatoire et pro-thrombotique.

Il a été montré dans l'état de la technique, qu'après invasion par un agent pathogène, la réponse de l'hôte se fait par la mise en jeu des systèmes d'immunité innée et d'immunité adaptative. L'immunité innée constitue la première ligne de défense, en libérant un grand nombre de chimiokines et cytokines (Aziz et al., 2013).

Les polynucléaires neutrophiles (PNN) sont des cellules de l'immunité innée. Ces cellules ont longtemps été considérées comme des cellules ayant pour seule fonction la phagocytose des pathogènes extracellulaires. Au cours des dernières années, l'évolution des connaissances sur la physiologie et la place des PNN a progressé dans un schéma plus complexe associant immunité innée, immunité adaptative, et hémostase (Mócsai, 2013). Si les PNN ont un rôle majeur dans la défense antibactérienne par l'intermédiaire de la phagocytose. Il a été démontré depuis le début des années 2000 qu'ils jouent également un rôle dans la protection vis-à-vis des germes intracellulaires (virus, mycobactéries) et dans la régulation de la réponse immunitaire adaptative, en particulier par l'interaction avec les lymphocytes B de la zone marginale au sein de la rate. Ainsi, les PNN sont capables de relarguer le contenu de leur noyau, ADN et histones, formant un maillage qui supporte des enzymes comme la myéloperoxidase (MPO) ou l'élastase. Ces structures, décrites pour la première fois en 2004, sont appelées NETs (Neutrophil Extracellular Traps) et sont capables de capturer des pathogènes dans leur réseau macromoléculaire (Brinkmann et al., 2004). Deux mécanismes de NETose sont décrits : l'un aboutit au suicide cellulaire et dure quelques heures, l'autre, la NETose "vitale", plus rapide permet la survie du PNN sous forme anucléée capable de phagocytose, fonction qui ne dépend pas de la synthèse de nouvelles protéines donc de la présence d'un noyau (Fuchs et al., 2007).

Les deux mécanismes s'accompagnent de modifications morphologiques des PNN décrites en microscopie électronique après induction de NETose in-vitro par du PMA ou phorbol-myristate-acetate (Brinkmann and Zychlinsky, 2012). D'abord, la chromatine se décondense, le noyau perd ses lobulations, le cytoplasme se dégranule et les NETs sont expulsés par la cellule. La voie de la NETose vitale semble impliquée au cours des infections par bactéries à Gram-positif. Les NETs sont relargués à partir des vésicules contenant la chromatine décondensée et les protéines anti-bactériennes.

Les NETs exercent une fonction antimicrobienne en piégeant bactéries et mycètes, mais pourraient également avoir un rôle dans la thrombino-formation. Ainsi, les NETs pourraient être un acteur majeur d'un nouveau concept appelé immunothrombose (Kessenbrock K, et al., 2015 ; Engelmann et Massberg, PMID 2013).

A ce jour, la rapidité du processus de NETose, sa focalisation au site de l'infection et la dégradation de l'ADN par les nucléases circulantes sont des obstacles à la détection directe *in vivo* dans le sang circulant. Toutefois, il existe des marqueurs indirects de NETose, notamment l'association de taux élevés de nucléosomes, d'histones citrullinées et de MPO circulants (Abrams et al., 2013),(Kessenbrock et al., 2009). La NETose a pu être associée par l'intermédiaire de ces marqueurs à différentes pathologies notamment thrombotiques, infectieuses, cancéreuses ou auto-immunes (Kessenbrock et al., 2009),(Demers et al., 2012).

Les NETs jouent donc non seulement un rôle dans les mécanismes de défense de l'hôte, mais participent aussi à l'interaction entre inflammation et coagulation durant les processus infectieux (Branzk and Papayannopoulos, 2013).

Il existe des procédés de détection des leucocytes et neutrophiles à l'aide de la cytométrie en flux, tels que décrits dans la demande de brevet américaine publiée sous le numéro US 2013/0101996, afin de déterminer, par exemple si l'organisme est dans un processus de défense de l'hôte.

Deux scores biologiques combinant les paramètres sanguins accessibles dans l'état de l'art actuel ont été proposés pour le diagnostic de CIVD. Une des difficultés de l'élaboration de scores réside dans le fait qu'il n'existe pas à ce jour de « définition biologique » consensuelle de la CIVD. Le premier test de détection décrit en 2001 émane de l'International Society for Thrombosis and Haemostasis (ISTH), le second de la Japanese Association for Acute Medicine (JAAM) en 2005 et revu en 2006.

Le score de l'ISTH est basé sur la présence d'une thrombopénie sévère, d'une diminution du taux de prothrombine (allongement du temps de Quick), d'une consommation du fibrinogène et d'une élévation des D-dimères, marqueurs de la fibrinolyse. Il permet de diagnostiquer les CIVD survenant au cours des pathologies néoplasiques, des traumatismes, de la pathologie obstétricale, mais apparait déficient au cours du choc septique en raison de l'importance du syndrome inflammatoire avec une thrombocytose, une élévation du fibrinogène et la présence de D-dimères. Il semble donc peu adapté à une détection de CIVD lors d'un choc septique.

Le score JAAM a été développé afin de diagnostiquer la CIVD lors de choc septique, et ne retient pas le fibrinogène comme critère diagnostic tout en utilisant des valeurs seuil plus « cohérentes » dans le cadre d'un sepsis pour les D-dimères et les plaquettes. Ces différences ont conduit à des discussions au niveau du comité CIVD de l'ISTH pour redéfinir l'entité CIVD en séparant coagulopathies obstétricales, traumatiques, septiques ou oncologiques.

Toutefois, ces scores ne peuvent être calculés, mis en œuvre qu'à partir d'échantillons particuliers et nécessitent un temps important pour l'obtention des résultats, générant ainsi un coût et un délai important dans la prise en charge.

En outre, il n'existe pas actuellement de procédé permettant de détecter de manière sure et systématique une CIVD. Il n'existe pas non plus de procédé et/ou de moyen permettant de prédire la survenue, par exemple à partir d'un échantillon biologique, d'une coagulation intravasculaire disséminée.

Il n'existe pas dans l'état de la technique de procédé permettant de déterminer chez un individu présentant un choc septique si celui-ci va déclencher/présenter dans un futur proche une coagulation intravasculaire disséminée. En revanche, il existe un procédé capable d'établir une prédiction d'un diagnostic négatif de CIVD chez un individu présentant un choc septique. Il s'agit notamment d'un score combinant 3 paramètres : La concentration de microparticules circulantes membranaires exposant la phosphatidylsérine et l'antigène CD105, la numération plaquettaire et le taux de prothrombine avec une spécificité de 71,2%, sensibilité de 71,0%, et une valeur prédictive négative de 93,1%. Ce procédé, même efficace, n'est pas technologiquement d'une mise en œuvre facile et rapide.

Il existe donc un réel besoin de trouver un procédé et/ou un moyen palliant ces défauts, inconvénients et obstacles de l'art antérieur, en particulier un procédé simple, rapide et efficace permettant de détecter la CIVD, afin d'améliorer le pronostic vital des individus et de réduire ainsi les coûts de sa prise en charge.

## Description de l'invention

La présente invention a pour but de surmonter les inconvénients de l'art antérieur en fournissant un procédé de prédiction et/ou de détection de la coagulation intravasculaire disséminée (CIVD) à partir d'un échantillon biologique comprenant une mesure de la fluorescence de neutrophiles avec un fluorochrome intercalant de l'ADN.

Dans la présente, par « marqueur biologique », on entend au moins un marqueur biologique choisi dans le groupe comprenant les neutrophiles, les plaquettes, les microparticules sanguines exposant la phosphatidylsérine.

La divulgation décrit une détection dudit au moins un marqueur biologique choisi peut être réalisée par tout procédé adapté connu de l'homme du métier.

La divulgation décrit une mesure du taux dudit au moins un marqueur biologique choisi peut être réalisée par tout procédé adapté connu de l'homme du métier.

La divulgation décrit une mesure de la concentration dudit au moins un marqueur biologique choisi peut être réalisée par tout procédé adapté connu de l'homme du métier.

Dans la présente par « neutrophiles » on entend les granulocytes neutrophiles ou polynucléaires neutrophiles (PNN) qui sont des cellules sanguines appartenant à la lignée blanche (leucocytes), qui ont donc un rôle dans le système immunitaire.

la mesure de la fluorescence de neutrophiles peut être effectuée par tout procédé adapté connu de l'homme du métier. Il peut s'agir par exemple d'un procédé utilisant un marqueur des neutrophiles, par exemple un marqueur fluorescent. La mesure de la fluorescence peut être réalisée par tout procédé adapté connu de l'homme du métier. Il peut s'agir par exemple d'un procédé en cytométrie en flux basé sur la reconnaissance de sondes fluorescentes et/ou de fluorochrome. Il peut s'agir par exemple d'un procédé comprenant un marquage des neutrophiles comprenant l'utilisation d'un fluorochrome. Il peut s'agit par exemple d'un procédé comprenant l'application d'un fluorochrome à un échantillon, par exemple sanguin après une étape de lyse des globules rouges par un premier réactif qui peut agir également sur la membrane des leucocytes en la perméabilisant permettant ainsi la fixation dudit fluorochrome.

La divulgation décrit par « fluorochrome » on entend un agent sans perméation membranaire à base ou non de polyméthine qui se lie aux acides nucléiques. Il peut s'agir avantageusement d'un fluorochrome qui ne diffuse dans le cytoplasme qu'au travers de la membrane, par exemple leucocytaire, perméabilisée et fixée. Les sondes fluorescentes peuvent être par exemple des intercalants s'insérant entre les bases de l'ADN ou des sondes spécifiques d'histones citrullinées par exemple des anticorps, ou des sondes spécifiques d'une conformation ou d'une modification post-traductionnelle des histones, des nucléosomes ou toute protéine de structure de l'ADN ou se fixant sur l'ADN en lien avec la décompaction de l'ADN ou par la NETose, ou d'une séquence nucléaire spécifiquement accessible au marquage au cours de la NETose, ou de sonde ciblant les cassures de l'ADN ou de sonde mesurant les phases précoces de l'apoptose comme les modifications membranaires plasmiques ou mitochondriales.

Selon l'invention le fluorochrome est un fluorochrome intercalant de l'ADN.

Selon l'invention la mesure de la fluorescence de neutrophile est effectuée avec un fluorochrome est un fluorochrome intercalant de l'ADN.

La divulgation décrit par exemple d'iodure de propidium, d'anticorps, par exemple anti-histone citrullinée, d'anticorps anti-ADN, de substrats fluorescents de l'activité de protéines liées à l'ADN décompacté comme l'élastase leucocytaire, la PR3, la myéloperoxidase, de mesure de modification de l'ADN par effet TUNEL, par cytométrie en flux, de mesure de l'Annexine V extra-membranaire, d'incorporation de DIOC6 (3,3'-dihexyloxacarbocyanine iodide).

Le fluorochrome et/ou sonde fluorescente peut être tout fluorochorme et/ou sonde fluorescente adapté(s) connu de l'homme du métier. Il peut s'agir, par exemple de fluorochrome et/ou sonde fluorescente disponible dans le commerce, par exemple commercialisés par la société Thermofisher sous les références commerciales Syto40, DIOC6(3) et Thiazol orange. L'homme du métier, de part ces connaissances générales saura choisir le fluorochrome et/ou la sonde adapté pour le marquage de neutrophiles.

En outre, l'homme du métier, de part ces connaissances générales, connait les différentes populations leucocytaires et saura les identifier, par exemple lors d'une cytométrie en flux, en fonction de leur granulométrie ou de leur taille.

Lorsque le procédé de mesure de la fluorescence de polynucléaires neutrophiles est un procédé utilisant un fluorochrome intercalant, par exemple un procédé de cytométrie en flux, il peut s'agir par exemple de Syto40, iodure de propidium et le cytomètre de flux Gallios (Beckman-Coulter) ou FACS-Canto (Becton-Dickinson) et/ou tout autre cytomètre en flux équipé par exemple de 3 lasers dont un violet (405 nm) et un bleu (488 nm) avec un réglage en gain logarithmique, avec par exemple la fluorescence du SYTO 40 mesurée sur un détecteur couplé avec le laser, violet (405 nm) et associé à un filtre passe-bande centré vers 450-460 nm, les autres lasers étant utilisés pour l'identification des leucocytes et des neutrophiles par exemple à l'aide d'anticorps anti CD45-FITC et CD49d.

Selon l'invention par « intensité de fluorescence » on entend tout signal mesuré à la longueur d'onde de réémission du fluorophore après excitation par exemple à 630 nm. L'intensité de fluorescence est proportionnelle à l'état de décompaction de l'ADN.

Dans la présente, la mesure de la fluorescense des neutrophiles peut être réalisée par tout dispositif connu de l'homme du métier adapté à la présente invention. Il peut s'agir par exemple d'un dispositif commercialisé par la société Sysmex, par la société Socimed, par la société Alere. Il peut s'agir par exemple de l'automate Sysmex XN.

Les inventeurs de la présente invention ont démontré que la mesure de la fluorescence des neutrophiles à partir d'un échantillon biologique, par exemple un échantillon sanguin, permet de déterminer la présence d'une CIVD chez un individu présentant un choc septique et/ou d'identifier un individu susceptible de développer une CIVD.

La présente invention a pour objet un procédé in-vitro de prédiction et/ou de détection de la coagulation intravasculaire disséminée (CIVD) à partir d'un premier échantillon biologique comprenant une étape de mesure de la fluorescence des neutrophiles. avec un fluorochrome intercalant de l'ADN. En outre, la présente invention a pour objet un procédé in-vitro de détection de la coagulation intravasculaire disséminée (CIVD) comprenant une mesure à partir d'un premier échantillon biologique de la fluorescence des neutrophiles.avec un fluorochrome intercalant de l'ADN.

Dans la présente, par « échantillon biologique », on entend tout échantillon obtenu à partir de mammifères, par exemple un mammifère choisi dans le groupe comprenant l'ordre Monotremata, Didelphimorphia, Paucituberculata, Microbiotheria, Notoryctemorphia, Dasyuromorphia, Peramelemorphia, Diprotodontia, Tubulidentata, Sirenia, Afrosoricida, Macroscelidea, Hyracoidea, Proboscidea, Cingulata, par exemple le tatou, Pilosa, Scandentia, Dermoptera, Primates, Rodentia, Lagomorpha, Erinaceomorpha, Soricomorpha, Chiroptera, Pholidota, Carnivora, Perissodactyla, Artiodactyla et Cetacea. Il peut s'agir par exemple d'un humain ou d'un animal. Il peut s'agir par exemple d'un animal d'élevage, d'un animal de compagnie, d'un animal en voie de disparition ou de tout autre animal.

Selon l'invention, l'échantillon biologique peut être un échantillon de sang.

Selon l'invention, l'échantillon biologique peut provenir d'un mammifère présentant un choc septique ou un risque de choc septique.

Selon l'invention, le procédé de détection peut comprendre en outre une étape de comparaison de l'intensité de la fluorescence mesurée avec une valeur de référence de fluorescence.

Selon l'invention l'intensité de fluorescence de référence peut être l'intensité de fluorescence des neutrophiles mesurée chez un sujet ou la moyenne de la valeur de la concentration mesurée chez un groupe de sujets sains de référence.

Dans la présente par « sujet sain de référence », on entend un mammifère, par exemple un être humain, n'ayant pas subi d'infection, de choc, d'hémorragie, de choc septique et/ou toute autre atteinte corporelle susceptible d'entrainer une coagulation intravasculaire disséminée telle que définie ci-dessus.

Selon l'invention par « groupe de sujets de référence » on entend un groupe permettant de définir une valeur de référence fiable. Il peut s'agir par exemple d'un groupe comprenant au moins 2 sujets de référence tel que définis ci-dessus, par exemple au moins 10, au moins 40, au moins 60, au moins 100 sujets de référence. Il peut s'agir par exemple d'un groupe comprenant de 30 à 500 sujets, de 40 à 200, de 45 à 110 sujets de référence.

Selon l'invention, la valeur de référence de fluorescence des neutrophiles $R_{Neut\,SFL}$ ou Rneutfl peut être également une valeur obtenue par analyse d'une courbe ROC obtenue, à partir d'analyse statique de valeurs obtenues à partir d'un sujet de référence et/ou groupe de sujets de référence. La valeur de référence peut être déterminée, par exemple obtenue via une courbe ROC tel que décrit dans Stiel et al. « Neutrophil Fluorescence: A New Indicator of Cell Activation During Septic Shock-Induced Disseminated Intravascular Coagulation. » Crit Care Med. 2016;44(11):e1132-e1136.

Selon l'invention, lorsque la fluorescence des neutrophiles est mesurée avec un dispositif commercialisé par la société Sysmex, par exemple de l'automate Sysmex XN, la valeur de référence de fluorescence des neutrophiles $R_{Neut\,SFL}$ ou Rneutfl obtenue via une courbe ROC tel que décrit dans Stiel et al. « Neutrophil Fluorescence: A New Indicator of Cell Activation During Septic Shock-Induced Disseminated Intravascular Coagulation. » Crit Care Med. 2016;44(11):e1132-e1136, peut être une fluorescence supérieure à 57 unités arbitraires.

Avantageusement, les inventeurs ont démontré que lorsque la valeur de fluorescence mesurée est supérieure à la valeur de référence, le procédé selon l'invention permet de détecter et/ou prédire une coagulation intravasculaire disséminée.

Avantageusement, les inventeurs de la présente invention ont également démontré que le procédé de détection de la CIVD comprenant la détermination/mesure de la fluorescence des neutrophiles permet une détection de la CIVD avec une sensibilité supérieure ou égale à 91 % et une spécificité supérieure ou égale à 81 %.

Selon l'invention, le procédé de prédiction et/ou de détection de la coagulation intravasculaire disséminée peut comprendre en outre une étape de mesure à partir d'un second échantillon biologique de la concentration $R0_{CD105}$ de microparticules membranaires porteuses du cluster de différenciation 105 (CD105) et exposant la phosphatidylsérine.

Dans la présente par « microparticules sanguines exposant la phosphatidylsérine » on entend des microparticules de la membrane plasmique exposant la phosphatidylsérine. Il peut s'agir par exemple de microparticules membranaires libérées, par exemple dans l'espace vasculaire à partir de cellules, par exemple des cellules endothéliales, des plaquettes, des monocytes, des granulocytes ou polynucléaires (neutrophiles, éosinophiles ou basophiles) ou de leurs précurseurs circulants, des cellules érythrocytaires, des lymphocytes ou d'origine non vasculaire exposant la phosphatidylsérine. Par exemple, les microparticules membranaires peuvent avoir une taille, c'est-à-dire un diamètre individuel, compris entre 50 nm et 1 $\mu$m et exposant la phosphatidylsérine. Par exemple, les microparticules membranaires peuvent porter une activité facteur tissulaire ou une activité fibrinolytique.

La mesure de la concentration de microparticules membranaires porteuses de Cluster de Différenciation peut être réalisée par tout procédé adapté connu de l'homme du métier. Il peut s'agir par exemple d'un procédé en cytométrie en flux basée sur la reconnaissance de sondes fluorescentes, par exemple des anticorps marqués, des sondes protéiques ou lipidiques, fixées ou non sur des billes, voire des sondes reconnaissant le matériel génétique inclus dans les microparticules comme les miRNA. Les sondes peuvent aussi être spécifiques de la PhosphatidylSérine (PhtdSer) par exemple l'annexine 5 ou tout autre dérivé d'annexine obtenu par génie moléculaire, par exemple les diannexines. Les sondes peuvent également, par exemple, reconnaitre des lipides de la membrane et leurs degrés de compaction, par exemple des sondes lipidiques à chaînes carbonées plus ou moins longues. Les sondes protéiques peuvent être par exemple des anticorps détectant les clusters de différentiation à la surface des microparticules ou détectant toute autre protéine ou enzyme hébergée à la surface des microparticules ou dans la microparticule comme l'actine, les métalloprotéinases, les récepteurs de protéines à activités enzymatiques, par exemple le facteur tissulaire ou uPAR les cytokines et interleukines. La mesure de la concentration des microparticules peut également être réalisée par dosage quantitatif des lipides totaux ou des protéines totales ou à l'aide de la technique Tunable Resistive Pulse Sensing. La mesure de la concentration de microparticules membranaire peut être également réalisée, par exemple, *via* un test prothrombinase tel que décrit dans Hugel B, Zobairi F, Freyssinet JM. Measuring circulating cell-derived microparticles. J Thromb Haemost. 2004 Oct;2(10):1846-7 [12]. Brièvement, dans ce procédé, les facteurs de coagulation du sang et la concentration de calcium sont déterminés en sorte que PhtdSer est le paramètre limitant dans la génération de thrombine soluble à partir de prothrombine exogène ajoutée dans le milieu réactionnel. Les résultats sont exprimés en nanomolaire d'équivalent PhtdSer (nM éq. PhtdSer) par référence à une courbe étalon établie avec des liposomes de composition connue et concentration tel que décrit dans Hugel B, Zobairi F, Freyssinet JM. « Measuring circulating cell-derived microparticles » J Thromb Haemost. 2004 Oct;2(10):1846-7 [12]. La mesure de la concentration des microparticules peut également être réalisée, par exemple, par mesure de l'activité facteur tissulaire. Brièvement le procédé utilise des facteurs de la coagulation spécifiques du complexe dit Tenase et de dose la formation de facteur Xa à partir de facteur X ajouté dans le milieu réactionnel tel que décrit dans Aupeix K, et al. « The significance of shed membrane particles during programmed cell death in vitro, and in vivo, in HIV-1 infection. » J Clin Invest 1997; 99(7): 1546-54. [13]. L'activité portée par les MP-FT+ est rapportée à une courbe étalon de concentration connue en facteur tissulaire. La concentration obtenue est exprimée en pmoles par litre d'activité facteur tissulaire. La mesure de la concentration de microparticules membranaires peut être également réalisée par exemple par une méthode chronométrique basée sur des billes recouvertes par un moyen de capture, par exemple un anticorps, permettant l'identification (phénotype) des microparticules et la mesure d'un temps de coagulation après ajout d'un plasma dépourvu de phospholipides. Dans cette méthode, le temps de coagulation est dépendant de la quantité de phospholipides anioniques (PhtdSer) apportés par les MPs spécifiquement captées au préalable par les billes. Une calibration du test chronométrique à partir d'une solution de liposomes de concentration connue afin peut être éventuellement effectuée et permet de convertir le résultat chronométrique (secondes) en activité procoagulante (nM eq. Phtd Ser). Il peut être réalisé par exemple à l'aide d'automates de la gamme STAR de l'entreprise Stago.

Selon l'invention, le second échantillon biologique est un échantillon biologique tel que défini ci-dessus.

Selon l'invention, le second échantillon biologique peut être identique ou différent du premier échantillon biologique.

Selon l'invention, le premier et second échantillons biologiques peuvent être un seul et même échantillon.

Selon l'invention, le premier et/ou le second échantillon biologique peut provenir d'un mammifère présentant un choc septique.

Selon l'invention, le procédé de prédiction et/ou de détection de la CIVD peut comprendre en outre une étape de comparaison de la concentration $R0_{CD105}$ mesurée avec une valeur de référence $R_{CD105}$.

Selon l'invention la valeur de référence $R_{CD105}$ peut être la valeur de la concentration de microparticules porteuses du cluster de différenciation CD105 mesurée chez un sujet ou la moyenne de la valeur de la concentration mesurée chez un groupe de sujets sains de référence.

Selon l'invention, le sujet sain de référence ou groupe de sujets sains de référence est tel que défini ci-dessus.

Selon l'invention, la valeur de référence $R_{CD105}$ peut être également une valeur obtenue par analyse d'une courbe ROC.

Selon l'invention, la valeur de référence $R_{CD105}$ peut être une concentration de microparticules porteuses du cluster de différenciation supérieure à 0,65 nM équivalent PhtdSer ou PS.

Avantageusement, les inventeurs ont démontré qu'une concentration de microparticules porteuses du cluster de différenciation CD105 mesurée supérieure à la valeur de référence selon le procédé de l'invention permet de détecter et/ou prédire une coagulation intravasculaire disséminée (CIVD).

Avantageusement, les inventeurs de la présente invention ont démontré que le procédé de détection de la CIVD comprenant en outre la détermination de la concentration $R0_{CD105}$ permet une détection avec une sensibilité supérieure ou égale à 69% et une spécificité supérieure ou égale à 48 %.

Selon l'invention, le procédé de prédiction et/ou de détection de la coagulation intravasculaire disséminée (CIVD) peut comprendre en outre une étape de mesure à partir d'un troisième échantillon biologique de la concentration plaquettaire R0Plt.

Dans la présente par « plaquettes » on entend des thrombocytes ou plaquettes sanguines formés par fragmentation des mégacaryocytes.

Selon l'invention, le troisième échantillon biologique est un échantillon biologique tel que défini ci-dessus.

Selon l'invention, le troisième échantillon biologique peut être identique ou différent du premier et/ou du second échantillon biologique.

Selon l'invention, le premier et troisième échantillons biologiques peuvent être un seul et même échantillon.

Selon l'invention, le second et troisième échantillons biologiques peuvent être un seul et même échantillon.

Selon l'invention, le premier, second et troisième échantillons biologiques peuvent être un seul et même échantillon.

Selon l'invention, la mesure de la concentration plaquettaire dans un échantillon peut être réalisée par tout procédé connu de l'homme du métier. Il peut s'agir par exemple des procédés décrits par McNair et al, JECT. 2015 ;47 :113-118 et/ou d'un procédé par exemple automatisé grâce à un compteur de type Coulter utilisant l'impédance électrique pour en dériver une concentration plaquettaire. En effet le passage de cellules en suspension dans un électrolyte modifiant la résistance électrique entre deux électrodes génère une variation d'impédance enregistrée sous forme d'impulsion corrélée au passage des cellules dont on cherche à établir la concentration (principe Coulter)

Selon l'invention, le procédé de prédiction et/ou de détection de la CIVD peut comprendre en outre une étape de comparaison de la concentration R0Plt mesurée avec une valeur de référence RPlt.

Selon l'invention la valeur de référence RPlt peut être la valeur de la concentration plaquettaire mesurée chez un sujet sain de référence ou la moyenne des valeurs de concentrations mesurées chez un groupe de sujets sains de référence.

Selon l'invention, le sujet sains de référence ou groupe de sujets sains de référence est tel que défini ci-dessus.

Selon l'invention, la valeur de référence RPlt peut être également une valeur obtenue par analyse d'une courbe ROC.

Selon l'invention la valeur de référence RPlt peut être un taux de plaquettes par exemple égale à 127 G/L

Avantageusement, les inventeurs de la présente invention ont démontré que le procédé de détection de la CIVD comprenant la détermination de la concentration RPlt permet une détection avec une sensibilité supérieure ou égale à 68% et une spécificité supérieure ou égale à 90 %.

Selon l'invention, le procédé de prédiction et/ou de détection de la coagulation intravasculaire disséminée (CIVD) peut comprendre en outre une étape de mesure du taux de prothrombine RoTP.

Selon l'invention, le procédé de prédiction et/ou de détection de la coagulation intravasculaire disséminée (CIVD) peut comprendre en outre une étape de mesure à partir d'un quatrième échantillon biologique du taux de prothrombine ROTP.

Selon l'invention, le quatrième échantillon biologique est un échantillon biologique tel que défini ci-dessus.

Selon l'invention, le quatrième échantillon biologique peut être identique ou différent du premier, du second échantillon et/ou du troisième échantillon biologique.

Selon l'invention, le premier et quatrième échantillons biologiques peuvent être un seul et même échantillon.

Selon l'invention, le second et quatrième échantillons biologiques peuvent être un seul et même échantillon.

Selon l'invention, le troisième et quatrième échantillons biologiques peuvent être un seul et même échantillon.

Selon l'invention, le premier, second et quatrième échantillons biologiques peuvent être un seul et même échantillon.

Selon l'invention, le premier, troisième et quatrième échantillons biologiques peuvent être un seul et même échantillon.

Selon l'invention, le second, troisième et quatrième échantillons biologiques peuvent être un seul et même échantillon.

Selon l'invention, le premier, second, troisième et quatrième échantillons biologiques peuvent être un seul et même échantillon.

Selon l'invention, la mesure du taux de prothrombine dans un échantillon peut être réalisée par tout procédé connu de l'homme du métier. Il peut s'agir par exemple du procédé décrit dans Delabranche et al., CCMED 2016 et/ou du procédé comprenant par exemple un dosage du facteur II par colorimétrie et/ou via l'utilisation d'un automate du type STAGO STA-R Evolution utilisant des réactifs commerciaux adaptés disponibles en routine.

Selon l'invention, le procédé de prédiction et/ou de détection de la CIVD peut comprendre en outre une étape de comparaison du taux de prothrombine ROTP mesurée avec une valeur de référence RTP.

Selon l'invention la valeur de référence RTP peut être la valeur du taux de prothrombine ROTP mesurée chez un sujet sain de référence ou la moyenne des valeurs de concentrations mesurées chez un groupe de sujets sains de référence.

Selon l'invention, le sujet sain de référence ou groupe de sujets sains de référence est tel que défini ci-dessus.

Selon l'invention, la valeur de référence ROTP peut être également une valeur obtenue par analyse d'une courbe ROC.

Selon l'invention la valeur de référence $R_{TP}$ peut être un taux de prothrombine inférieur à 58%.

Avantageusement, les inventeurs ont démontré que lorsque la valeur du taux de prothrombine mesurée est inférieure à la valeur de référence, le procédé selon l'invention permet de détecter et/ou prédire une coagulation intravasculaire disséminée (CIVD).

Avantageusement, les inventeurs de la présente invention ont également démontré que le procédé de détection de la CIVD comprenant la détermination de la concentration $Ro_{TP}$ permet une détection avec une sensibilité supérieure ou égale à 73 et une spécificité supérieure ou égale à 60%.

Avantageusement, les inventeurs de la présente invention ont également démontré que le procédé de détection de la CIVD comprenant la détermination de la combinaison de la fluorescence et du CD105, des plaquettes et du taux de prothrombine permet une détection avec une sensibilité supérieure ou égale à 76% et une spécificité supérieure à 86%.

En d'autres termes, le procédé selon l'invention comprenant, à partir d'au moins un échantillon biologique, la mesure de la fluorescence des neutrophiles, par exemple en utilisant des automates Sysmex XN, la mesure de la concentration plaquettaire, du taux de prothrombine et de microparticules membranaires comprenant le cluster de différenciation 105 (CD105) permet une détection avec une sensibilité supérieure ou égale à 76% et une spécificité supérieure ou égale à 86%.

Avantageusement, les inventeurs de la présente invention ont également démontré que le procédé de détection de la CIVD comprenant la détermination de la combinaison de la fluorescence des neutrophiles, de la numération plaquettaire et du taux de prothrombine permet une détection avec une sensibilité supérieure ou égale à 66 et une spécificité supérieure ou égale à 85%

Dans la présente la valeur de fluorescence des neutrophiles mesurée R0Neut SFL est indépendamment désignée ROneutfl, la valeur Vneutfl peut être également désignée Vneutsfl et la valeur de référence RNeut SFL est indépendamment désignée RNeutfl.

La présente invention a également pour objet un procédé in-vitro de détection et/ou de suivi de l'évolution d'une coagulation intravasculaire disséminée (CIVD) à partir d'un échantillon biologique comprenant les étapes suivantes :

a. mesure de la fluorescence des neutrophiles $R_{0neutfl}$ avec un fluorochrome intercalant de l'ADN

b. comparaison de la valeur mesurée de fluorescence des neutrophiles ROneutfl avec une valeur de référence RNeutfl

c. mesure de la concentration $R_{0CD105}$ de microparticules membranaire comprenant le cluster de différenciation 105 (CD105),

d. comparaison de la valeur mesurée $R_{0CD105}$ avec une valeur de référence $R_{CD105}$

e. mesure de la concentration plaquettaire RoPlt

f. comparaison de la valeur mesurée RoPlt avec une valeur de référence RPlt

g. mesure du taux de prothrombine RoTP

h. comparaison de la valeur mesurée RoTP avec une valeur de référence RTP

i. affectation d'une valeur fluorescence des neutrophiles Vneutfl égale à 1 si ROneutfl est supérieure à la valeur $R_{neutfl}$ ou d'une valeur Vneutfl égale à 0 si ROneutfl est inférieure à la valeur $R_{neutfl}$

j. affectation d'une valeur $V_{CD105}$ égale à 1 si $R_{0CD105}$ est supérieur à la valeur $R_{CD105}$ ou d'une valeur CD 105 égale à 0 si $R_{0CD105}$ est inférieur à la valeur $R_{CD105}$

k. affectation d'une valeur $V_{Plq}$ égale à 1 si RoPlt est inférieur à la valeur RPlt ou d'une valeur $V_{Plq}$ égale à 0 si RoPlt est supérieur à la valeur RPlt

l. affectation d'une valeur $V_{TP}$ égale à 1 si RoTP est inférieur à la valeur RTP ou d'une valeur $V_{TP}$ égale à 0 si RoTP est supérieur à la valeur RTP

m. calcul du score S1 selon la formule suivante :

$$S1 = \varepsilon + Vneutfl \times \alpha + V_{CD105} \times \beta + V_{plaquettes} \times \Omega + V_{TP} \times \theta$$

dans laquelle $\varepsilon$ est compris de -0,708 à 2,280, par exemple égal à 0,786, $\alpha$ est compris de 0,152 à 1,928, par exemple égal à 1,040, $\beta$ est compris entre 0,154 à 1,549, par exemple égal à 0,851, $\Omega$ est compris entre -0,016 à - 0,005, par exemple égal à -0,010, $\theta$ est compris entre -0,036 à 0,001, par exemple égal à -0,017.

En outre, les inventeurs ont montré que lorsque la valeur de S1 est supérieure à -0,47 le procédé permet avantageusement une prédiction avec une sensibilité supérieure ou égale à 76% et une spécificité supérieure ou égale à 86%.

En d'autres termes les scores obtenus peuvent être corrélés avec le devenir du mammifère dont l'échantillon biologique provient.

Selon l'invention le procédé peut comprendre alternativement et/ou additionnellement au calcul du score S1, une étape n. de calcul du score S2 comme suit :

n. Calcul du score S2 selon la formule suivante :

$$S2 = \varepsilon + Vneutfl \times \alpha + V_{plaquettes} \times \Omega + V_{TP} \times \theta$$

dans laquelle $\varepsilon$ est compris de 0,381 à 3,070, par exemple égale à 1,726, $\alpha$ est compris de 0,209 à 1,910, par exemple égale à 1,060, $\Omega$ est compris entre -0,015 à -0,005, par exemple égale à -0,10, et $\theta$ est compris entre -0,040 à -0,004, par exemple égale à -0,022

En outre, les inventeurs ont démontré que lorsque la valeur de S2 est supérieure à 0,16 le procédé permet avantageusement une prédiction d'une CIVD avec une sensibilité supérieure ou égale à 66% et une spécificité supérieure ou égale à 95%

En d'autres termes, les inventeurs ont clairement démontré que les scores obtenus peuvent être corrélés avec le devenir du mammifère dont l'échantillon biologique provient et permettent avantageusement de détecter une CIVD.

La présente décrit également un procédé de détection et/ou de suivi de l'évolution d'une coagulation intravasculaire disséminée (CIVD) à partir d'un échantillon biologique comprenant les étapes suivantes :

a. mesure de la concentration $R_{0CD105}$ de microparticules membranaire comprenant le cluster de différenciation 105 (CD105),

b. comparaison de la valeur mesurée $R_{0CD105}$ avec une valeur de référence $R_{CD105}$

c. mesure de la concentration plaquettaire RoPlt

d. comparaison de la valeur mesurée RoPlt avec une valeur de référence RPlt

e. mesure du taux de prothrombine RoTP

f. comparaison de la valeur mesurée RoTP avec une valeur de référence RTP

g. affectation d'une valeur $V_{CD105}$ égale à 1 si $R_{0CD105}$ est supérieur à la valeur $R_{CD105}$ ou d'une valeur $V_{CD105}$ égale à 0 si $R_{0CD105}$ est inférieur à la valeur $R_{CD105}$

h. affectation d'une valeur $V_{Plq}$ égale à 1 si RoPlt est inférieure à la valeur RPlt ou d'une valeur $V_{Plq}$ égale à 0 si RoPlt est supérieure à la valeur RPlt

i. affectation d'une valeur $V_{TP}$ égale à 1 si RoTP est inférieure à la valeur RTP ou d'une valeur $V_{TP}$ égale à 0 si RoTP est supérieure à la valeur RTP

j. calcul du score S3 selon la formule suivante :

$$S3 = \varepsilon + \alpha \times V_{CD105} + \beta \times V_{Plq} + \gamma \times V_{TP}$$

dans laquelle $\varepsilon$ est compris entre 0,686 et 2,830, par exemple égale à 1,758, $\alpha$ est compris entre 0,180 et 0,785, par exemple égale à 0,483, $\beta$ est compris entre -0,018 et -0,009, par exemple égale à -0,014, et $\gamma$ compris entre -0,027 et 0,001, par exemple égale à -0,013.

Les inventeurs ont démontré que lorsque la valeur de S3 est supérieure à 0,03 le procédé permet avantageusement une prédiction de CIVD avec une sensibilité supérieure ou égale à 67% et une spécificité supérieure ou égale à 94%.

En d'autres termes les scores obtenus peuvent être corrélés avec le devenir du dont l'échantillon biologique provient

En d'autres termes les scores obtenus peuvent être corrélés avec le devenir du dont l'échantillon biologique provient

La présente décrit également un examen de détection de la coagulation intravasculaire disséminée (CIVD) selon l'invention comprenant des moyens de mesure de la Fluorescence des neutrophiles et de la concentration de neutrophiles.

Les moyens de mesure de la fluorescence et/ou de la concentration de neutrophiles sont tel que définis ci-dessus.

La présente décrit également un procédé in-vitro de détection de la coagulation intravasculaire disséminée (CIVD) comprenant une étape d'analyse morphologique des neutrophiles sur un frottis sanguin.

le procédé peut comprendre en outre une étape d'analyse morphologique des neutrophiles, par exemple sur un frottis sanguin.

Selon l'invention, le procédé de prédiction et/ou de détection de la CIVD peut comprendre en outre une étape de comparaison de la morphologie des neutrophiles, par exemple sur un frottis sanguin, avec la morphologie de neutrophiles observée, par exemple à partir d'un frottis sanguin, d'un sujet ou d'un groupe de sujets sains de référence.

Selon l'invention, le sujet sain de référence ou groupe de sujets sains de référence est tel que défini ci-dessus.

Selon l'invention, l'analyse morphologique peut être réalisée à partir d'un frottis sanguin. Selon l'invention, un frottis sanguin peut être obtenu par tout procédé adapté connu de l'homme du métier. Il peut s'agir par exemple du procédé décrit dans Stiel et al « Neutrophil Fluorescence: A New Indicator of Cell Activation During Septic Shock-Induced Disseminated Intravascular Coagulation. » Crit Care Med. 2016;Nov;44(11):e1132-e1136 permettant notamment une analyse par observation visuelle, grâce à un microscope optique suite à une coloration May-Grünwald-Giemsa ou Wright réalisée sur lame.

Selon l'invention, l'étape d'analyse ou de comparaison de la morphologie des neutrophiles peut comprendre l'observation d'au moins un paramètre choisi dans le groupe comprenant la condensation chromatienne des neutrophiles, les granules des neutrophiles et les vacuoles des neutrophiles.

Selon l'invention, l'étape d'analyse ou de comparaison de la morphologie des neutrophiles peut comprendre l'observation d'au moins deux paramètres choisis dans le groupe comprenant la condensation chromatienne des neutrophiles, les granules des neutrophiles et/ou les vacuoles des neutrophiles.

Selon l'invention, l'étape d'analyse ou de comparaison de la morphologie des neutrophiles peut comprendre l'observation de la condensation chromatinienne des neutrophiles, des granules des neutrophiles et des vacuoles des neutrophiles.

Selon l'invention, la chromatine des neutrophiles peut être condensée ou décondensée. L'homme du métier de part ces connaissances générales sait déterminer la condensation ou la décondensation de la chromatine de neutrophiles.

Selon l'invention, l'observation des granules des neutrophiles peut comprendre la détermination du nombre et/ou de la densité desdites granules des neutrophiles. L'homme du métier par ces connaissances générales connait l'aspect morphologique et ladensité normales des granules des neutrophiles.

Selon l'invention, l'observation des vacuoles peut comprendre la détermination de la présence ou l'absence de vésicules de neutrophiles sur ledit frottis. L'homme du métier de part ces connaissances générales sait déterminer la présence ou l'absence de vacuoles sur des neutrophiles.

Selon l'invention, le procédé peut comprendre en outre une étape de comparaison entre la morphologie, par exemple anormale, des neutrophiles issus d'un échantillon biologique et la morphologie normale des neutrophiles chez un sujet sain et l'établissement d'un score cytologique d'anormalité.

Selon l'invention, l'analyse morphologique peut être réalisée par exemple par échantillon biologique sur 1 à 1000 neutrophiles, par exemple sur 1 à 100 neutrophiles. Par exemple, lorsque l'analyse morphologique est effectuée sur un frottis sanguin, elle peut être réalisée sur 1 à 100 neutrophiles.

La présente décrit également pour objet un procédé de détection et/ou de suivi de l'évolution d'une coagulation intravasculaire disséminée (CIVD) à partir d'un échantillon biologique comprenant en outre les étapes suivantes :

- observation de la condensation chromatinienne des neutrophiles,
- observation du nombre et/ou de la densité des granules des neutrophiles,
- observation des vacuoles des neutrophiles,

D'autres avantages pourront encore apparaître à l'homme du métier à la lecture des exemples ci-dessous, illustrés par les figures annexées, donnés à titre illustratif.

**Brève description des figures**

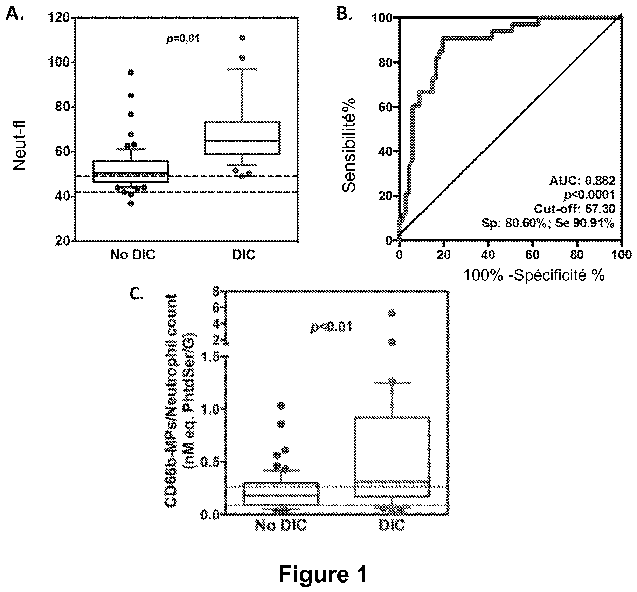

La Figure 1 représente une fluorescence des polynucléaires neutrophiles (PNN) (NEUT-FL) plus élevée et significativement associée à la CIVD. Les résultats sont représentés par des boîtes à moustaches rouges (CIVD) ou bleue (absence de CIVD). La ligne horizontale dans la boîte correspond à la médiane. Les limites, supérieure et inférieure, de la boîte correspondent respectivement aux 25ème et 75ème percentiles et les barres supérieure et inférieure aux 10ème et 90ème percentiles. Les lignes pointillées correspondent à des valeurs de référence. Les analyses statistiques représentent les différences entre les mesures répétées entre les patients avec ou sans CIVD. A. NEUT-FL chez 100 patients en choc septique. B. Courbe ROC (Receiver-operating characteristic) de NEUT-FL au cours du choc septique. C. Microparticules neutrophiliques rapportées au taux de neutrophiles chez 100 patients en choc septique.

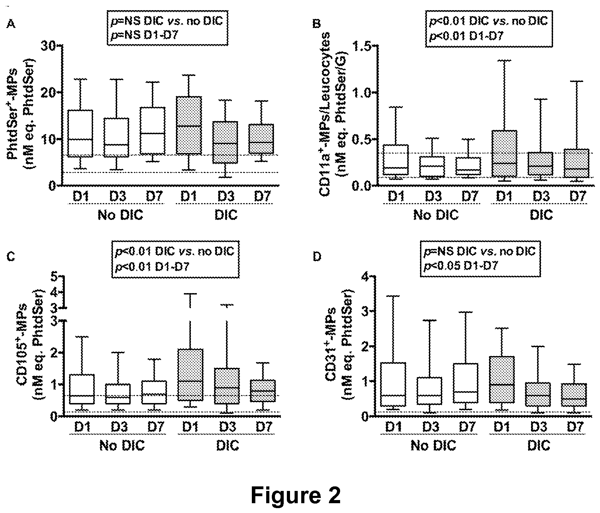

La Figure 2 représente un diagramme en bâton de la concentration moyenne des microparticules membranaires plasmatiques en phosphatidylSérine équivalent (ordonnée) en fonction du temps lors d'une CIVD (en gris) ou non (blanc). Les lignes en pointillées représentent les valeurs normales. Les analyses statistiques représentent les différences des mesures répétées entre les sujets avec une CIVD ou non en fonction du temps.

La Figure 2A représente un diagramme en bâton de la concentration moyenne des microparticules membranaires en phosphatidylsérine équivalent (ordonnée) lors d'un choc septique (en gris) ou non (blanc). Les résultats représentés sont la concentration totale de microparticules membranaires (PhtdSer-microparticules). Figure 2B représente les concentrations des microparticules membranaires dérivées des leucocytes (CD11a-microparticules) rapportées aux leucocytes circulants et Figure 2C des microparticules membranaires dérivées des cellules endothéliales CD105-microparticules et Figure 2D des microparticules membranaires dérivées des cellules endothéliales CD31-microparticules.

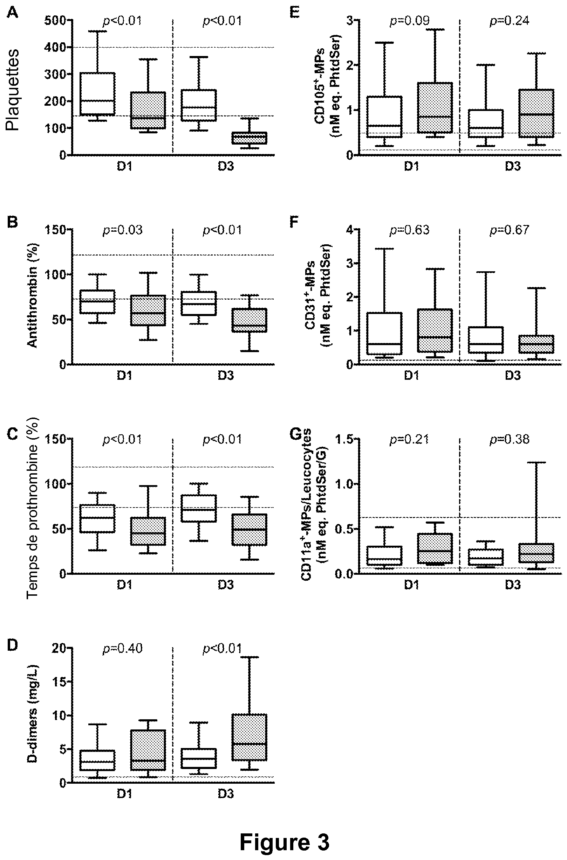

La Figure 3 représente des diagrammes en bâton des résultats obtenus en fonction de la présence d'une CIVD (gris) ou pas de CIVD (blanc). Les lignes en pointillés représentent les valeurs normales. L'ordonné représente la concentration des leucocytes en G/L, des plaquettes en G/L, le rapport microparticules membranaires portant le CD11a sur le nombre de leucocytes en nM/G, le taux de prothrombine en %, le taux d'antithrombine en %, la concentration en D-Dimères en mg/L, les taux circulants de microparticules CD-105 et microparticules CD-31. L'abscisse représente la condition et le jour de mesure : 1er (D1), 3ème (D3). Les analyses statistiques représentent les différences des mesures répétées entre les sujets avec une CIVD ou non en fonction du temps.

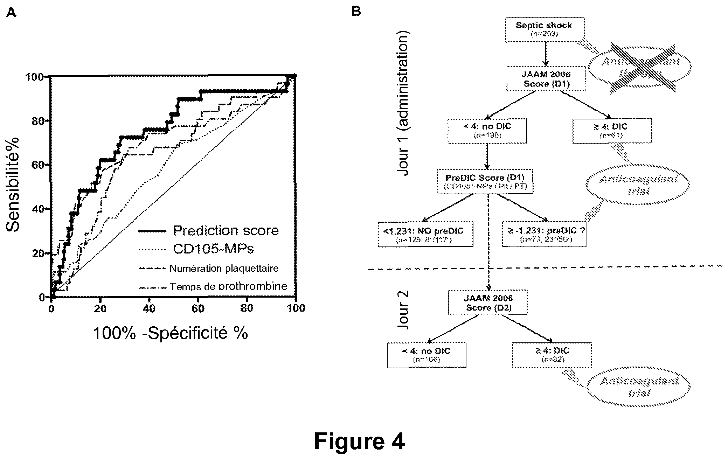

La Figure 4 représente en A. les courbes ROC de CD-105 - microparticules, le taux de prothrombine, numération plaquettaire et le score prédictif de CIVD, en B, l'arbre décisionnel des patients en option de traitement. N+ renvoie au nombre de patients avec CIVD, N- est le nombre de patients sans CIVD.

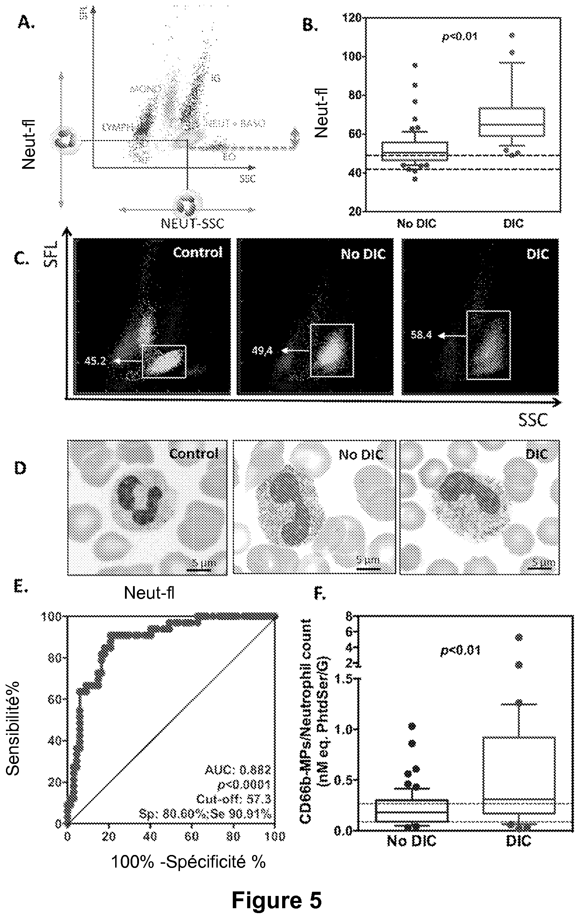

La Figure 5 complète la figure 4, avec en A la représentation des fenêtres enregistrées par le cytomètre automatique et utilisées pour l'analyse du paramètre NEUT-FL. En C les variations des « scattergrammes » chez les sujets contrôles, ou en sepsis sans ou avec DIC. En D. Les modifications morphologiques observées sur des frottis sanguins après coloration MGG.

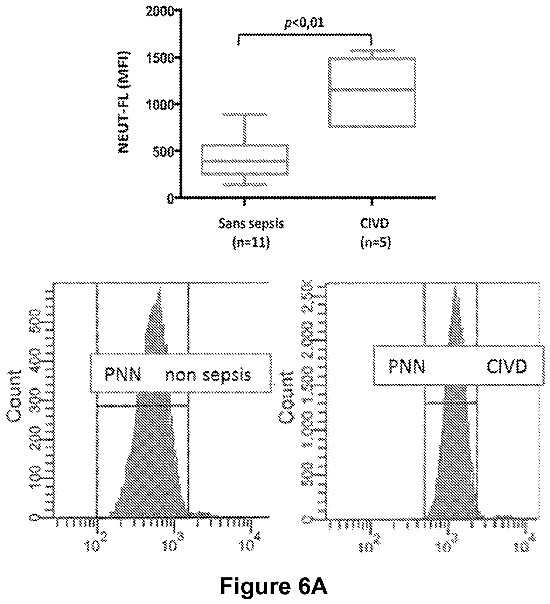

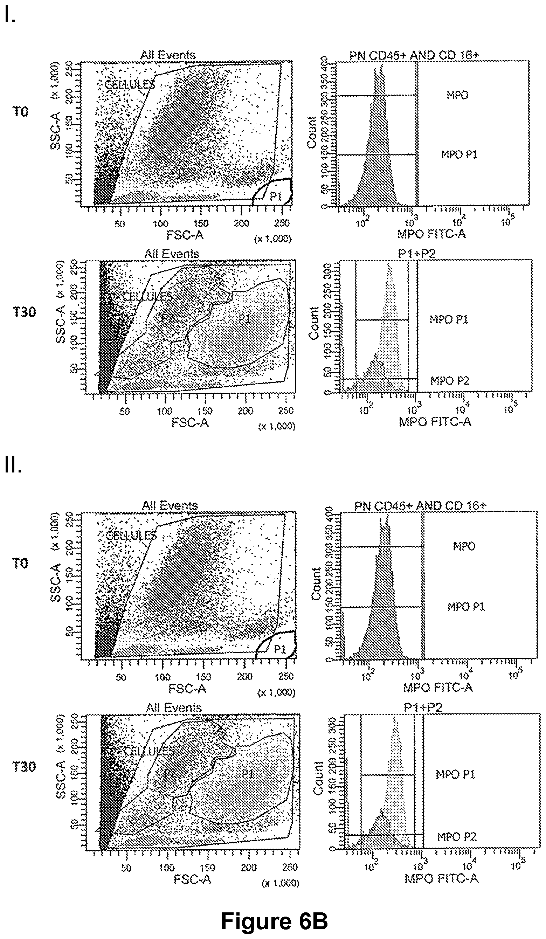

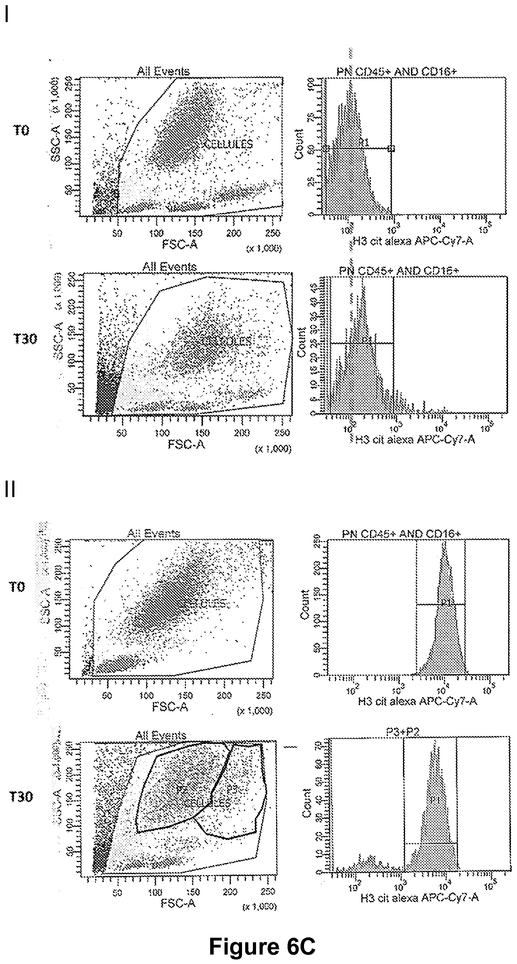

La Figure 6 représente les résultats obtenus par cytométrie conventionnelle dans les expériences décrites dans l'exemple 4.

La figure 6A représente les diagrammes en boîte à moustaches de la moyenne de fluorescence de neutrophiles (« NEUT-FL (MFI) ») marqués avec l'agent intercalant Syto40 chez des patients septiques en CIVD (« CIVD ») (n=5) et chez des sujets non septiques (« non sepsis ») (n=11) ainsi que les histogrammes de fluorescences correspondants (« PNN non sepsis » et « PNN CIVD » respectivement).

La figure 6B et 6C présente les scattergrammes (SSC/FSC) de l'ensemble des cellules et les diagrammes de fluorescence des neutrophiles obtenus après marquage intracellulaire et extracellulaire des neutrophiles pour la détection des NETs dans les échantillons sanguins de sujets non septiques et après déclenchement de la Netose par un agent exogène (ionomycine). Les deux marqueurs de NETs étudiés sont la myeloperoxydase et les histones H3 citrullinées. P1, P2 , P3 indiquent des sous-populations de neutrophiles identifiées sur les scattergrammes SSC et FSC grâce au signal de fluorescence associé au marquage (couleurs correspondantes entre scattergrammes et hstogrammes).

La figure 6B I représente les scattergrammes (SSC/FSC) de l'ensemble des cellules et les diagrammes de fluorescence des neutrophiles avant (T0) et après marquage de myeloperoxydase à la surface des neutrophiles en Netose après incubation 30 minutes avec ionomycine (T 30)

La figure 6B II représente les scattergrammes (SSC/FSC) de l'ensemble des cellules et les diagrammes de fluorescence des neutrophiles avant (T0) et après marquage intracellulaire de myeloperoxydase des neutrophiles en Netose après incubation 30 minutes avec ionomycine (T 30)

La figure 6 C I représente les scattergrammes (SSC/FSC) de l'ensemble des cellules et les diagrammes de fluorescence des neutrophiles avant (T0) et après marquage d'histones citrullinées à la surface des neutrophiles en Netose après incubation 30 minutes avec ionomycine (T 30)

La figure 6 C II représente les scattergrammes (SSC/FSC) de l'ensemble des cellules et les diagrammes de fluorescence des neutrophiles avant (T0) et après marquage intracellulaire d'histones citrullinées des neutrophiles en Netose après incubation 30 minutes avec ionomycine (T 30)

## EXEMPLE

### Exemple 1. Utilisation d'un score combinant CD105$^+$-MPs, le taux de prothrombine et la numération plaquettaire
Résumé :

**Objectifs:** la stratification des patients en choc septique est insuffisante et peut entraîner une allocation inappropriée de traitement dans les essais cliniques randomisés (ECR), en particulier en ce qui concerne les traitements anticoagulants. Nous montrons que les microparticules dérivées de l'endothélium CD-105 sont les biomarqueurs pertinents de la coagulation intravasculaire disséminée (CIVD) dans le choc septique. Étude prospective observationnelle chez les patients en choc septique hospitalisés dans quatre unités de réanimation.

Patients et méthodes: 265 patients atteints de choc septique consécutivement hospitalisés, la CIVD a été diagnostiquée selon JAAM score 2006.

**Principaux résultats:** 259 patients ont été analysés. 61 avaient une CIVD à l'admission (CIVD-J1) et 32 une CIVD développée durant les 24 premières heures après l'admission (CIVD-J2). Plusieurs modèles de régression logistique ont confirmé que les MP dérivées des cellules endothéliales ont été associées à la CIVD: CD105+-MPs (OR 2,13) et CD31+-MPs (OR 0,65) (p <0,05). En outre, le rapport CD11a-microparticules sur leucocytes témoigne de l'activation des leucocytes (OR 1,59, p <0,05). La prédiction de la CIVD a également été analysée après exclusion des patients CIVD-J1. Une nouvelle analyse par régression logistique multiple a démontré l'association de CD105-MPs (> 0,60 nM éq. PhtdSer, OR 1,67, p <0,01), numération plaquettaire (≤127 G / L ou 0,99, p <0,01) et le temps de prothrombine (≤58%, OR 0,98, p <0,05) avec la CIVD. Un score combinant ces marqueurs à l'admission est prédictif de l'absence de CIVD (AUC 72,9%, une spécificité de 71,2%, une sensibilité de 71,0%, avec une valeur prédictive négative de 93,1% et une valeur prédictive positive de 31,0%).

## Patients et méthodes

### Patients

250 patients consécutifs âgés de 18 à 85 ans ont été inclus. Tous les patients remplissaient les critères de choc septique à l'admission. Selon le score JAAM 2006 [10], 40% des patients présentent une CIVD précoce définie par un score JAAM ≥4 à J1 ou à J2. Les caractéristiques des patients sont résumées dans le tableau 1. Les patients présentant une CIVD étaient plus gravement malades avec des scores de gravité SOFA (Sequential Organ Failure Assesment Score) et SAPS2 (Simplified Acute Physiology Score) ainsi qu'une mortalité plus élevée statistiquement dans le groupe CIVD. Les numérations plaquettaires et des neutrophiles sont significativement différents entre les deux groupes.

**Tableau 1.** Caractéristiques des patients

|  | Total (259) | CIVD (-) (166) | CIVD (+) (93) | *p* value |
|---|---|---|---|---|
| **Charactéristiques** |  |  |  |  |
| Hommes - N (%) | 162 (62,5) | 106 (63,9) | 56 (60,2) | 0,561 |
| Age - an | 66,8±13,3 | 67,2±13,6 | 66,1±12,8 | 0,523 |
| SAPS II | 57,1±19,0 | 52,9±17,1 | 64,6+19,9 | <0,001 |
| Mortalité jour 28 - N (%) | 89 (34,4) | 47 (28,3) | 42 (45,2) | <0,001 |
| **Organe affecté** |  |  |  |  |
| SOFA dès l'admission | 11,3±3,1 | 10,2±2,8 | 13,2±2,6 | <0,001 |
| Troubles respiratoires - N (%) | 221 (85,3) | 137 (82,5) | 84 (90,3) | 0,089 |
| Atteinte aiguë rénale - N (%) | 144 (55,6) | 69 (41,6) | 75 (80,6) | <0,001 |
| Atteinte hépatique - N (%) | 47 (18,1) | 19(11,4) | 28 (30,1) | <0,001 |
| **Localisation de l'infection** |  |  |  | **0,589** |
| Poumons | 100 (38,6) | 65 (39,2) | 35 (37,5) | - |
| Urinaire | 28 (10,8) | 17 (10,2) | 11 (11,8) | - |
| Abdominale | 27 (10,4) | 13 (7,8) | 14 (15,1) | - |
| Bactériémie | 54 (20,8) | 32 (19,3) | 22 (23,7) | - |
| Inconnue | 34 (13,1) | 22 (13,2) | 12 (13,0) | - |
| **Soin** |  |  |  |  |
| Epuration extra-Rénale - N (%) | 89 (34,4) | 40 (24,1) | 49 (52,7) | <0,001 |
| Adrénaline - N (%) | 43 (16,6) | 15 (9,0) | 28 (30,4) | <0,001 |
| Plasma frais congelé - N (%) | 26 (10,0) | 7 (4,2) | 19 (20,4) | <0,001 |
| Plaquettes concentrées - N (%) | 26 (10,0) | 4 (2,4) | 22 (23,7) | <0,001 |
| Héparine - N (%) | 191 (73,7) | 124 (74,7) | 67 (72,0) | 0,641 |
| HBPM - N (%) | 59 (22,8) | 44 (26,5) | 15 (16,1) | 0,056 |

### Prélèvements sanguins :

Des tubes ont été prélevés pour les analyses nécessaires aux soins des patients, dont un tube EDTA (Vacutainer™, Becton Dickinson, Le Pont de Claix, France) utilisé pour la réalisation de la numération formule, sanguine et détection de la fluorescence liée à la décompaction de l'ADN. Quinze millilitres de sang ont été prélevés dès le diagnostic de choc septique posé puis au troisième (J3) et septième jour (J7) sur tube citraté (0,109M, Vacutainer™, Becton Dickinson, Le Pont de Claix, France) et sur tube sec (Vacutainer™, Becton Dickinson, Le Pont de Claix, France). Les tubes ont immédiatement été centrifugés deux fois à 2500g pendant 15 minutes pour obtenir du plasma pauvre en plaquettes et du sérum et les échantillons sont immédiatement congelés à -80°C. Les prélèvements sanguins ont été effectués après mise en place d'un cathéter artériel destiné à la surveillance invasive de la pression artérielle et aux multiples prélèvements sanguins rendus nécessaires par l'état du patient et n'ont pas entraîné de ponction supplémentaire. Il n'y a pas eu de prélèvement sanguin supplémentaire pour l'étude. La voie d'abord la plus fréquemment utilisée était au niveau de l'artère radiale. Les six volontaires sains ont été prélevés par prélèvement sanguin veineux périphérique.

## Numérations et formules sanguines

Les numérations et les formules sanguines ont été effectuées par à l'aide d'un cytomètre de en flux automatisé (XN20 Sysmex® Corporation, Kobe Japon) après acheminement des tubes de sang au laboratoire d'hématologie, selon le circuit hospitalier interne habituel de traitement des bilans. Les formules numérations ont été rendues à partir des canaux de fluorescence et après un marquage utilisant 2 réactifs (description selon Sysmex® Corporation): le premier réactif lyse les globules rouges et agit sur la membrane des leucocytes en la perméabilisant. Le second réactif lié à un fluorochrome à base de polyméthine se lie aux acides nucléiques. Il s'agit d'un agent non perméant appliqué après la fixation des cellules. Les différents leucocytes ont été ensuite identifiés en fonction de leur fluorescence à l'aide des capteurs de diffusion latérale et frontale et de la lumière à une longueur d'onde de 633nm.

## Hémostase :

Les bilans d'hémostase ont été effectués sur l'automate STA-R (marque déposée) Evolution (Stago, Asnières, France) à l'aide de réactifs de routine. Les taux de prothrombine (TP), le temps de céphaline activé (TCA), le taux de facteur V (FV), le taux de fibrinogène, les D-Dimères (DD) et l'antithrombine (AT) ont ainsi été mesurés pour chaque malade.

Il n'y a pas de « gold standard » pour définir la CIVD. Nous avons décidé de définir le diagnostic de CIVD conformément au score JAAM 2006[10]. La CIVD précoce a été définie par un score JAAM (Japanese Association for Acute Medicine) supérieur ou égal à 4 pendant les 48 premières heures du choc.

**Tableau 2.** Le score JAAM: tableau d'après *Gando*

| Paramètres biologiques | JAAM |
|---|---|
| Syndrome de réponse inflammatoire systémique défini par $\geq$ 2 des conditions suivantes remplies<br>* température<36°C ou > 38°C<br>* fréquence cardiaque > 90/min<br>* fréquence respiratoire > 20/min ou PaCO2 < 32mmHg<br>* leucocytose > 12000/mm3, <4000/mm3 ou présence de forme immatures circulantes (>10% des cellules) | [0] < 3<br>[1] $\geq$ 3 |
| Plaquettes (G/L) | [0] > 120<br>[1] 80-119 ou 30%<br>[3] < 80 ou 50% |
| Taux de prothrombine (%) | [0] > 64<br>[1] < 64 |
| D-dimères $\mu$g/mL) | [0] <5,0<br>[1] 5,0 - 15,0<br>[3] > 15,0 |
| Diagnostic de CIVD | Score $\geq$ 4/8 |

## Dosage des microparticules :

L'activité pro-coagulante des MPs a été dosée par test fonctionnel prothrombinase sur plaque multi-puits streptavidinée (Roche®, France) par capture sur Annexine V ou grâce à des anticorps spécifiques de l'origine cellulaire pour le phénotypage microparticulaire. Ceci a été réalisé aux différents temps de prélèvement.

La méthode de dosage fonctionnel des MPs mise au point par l'équipe utilise un test enzymatique prothrombinase effectué dans une plaque multi-puits. Le dosage est réalisé après capture des MPs sur de l'Annexine V biotinylée insolubilisée au fond des puits recouverts de streptavidine. Le test utilise, d'une part, la haute affinité de l'Annexine V (AV) pour la Phosphatidylsérine (PhtdSer) exprimée à la surface des MPs, et d'autre part celle de la biotine pour la streptavidine. La streptavidine est fixée de manière covalente au fond des puits et l'AV biotinylée se fixe à la streptavidine. Pour le phénotypage des MPs, l'AV est remplacée par différents anticorps (Ac) monoclonaux biotinylés spécifiques de différentes cibles spécifiques de l'origine cellulaire des MPS. Nous utilisons ainsi un Ac anti-CD105 (endogline) - (R&D Systems, Mineapolis, USA) pour caractériser les MPs d'origine endothéliale, un Ac anti-CD11a (Leinco Technologies, St Louis, USA) pour caractériser les MPs d'origine leucocytaire, et un Ac anti-CD66b -CEACAM8- (BD Pharmingen, Franklin Lakes, USA) pour caractériser spécifiquement les MPs d'origine neutrophilique. Les normes chez des volontaires sains ont été établies au cours de travaux préalables (Stiel et al « Neutrophil Fluorescence: A New Indicator of Cell Activation During Septic Shock-Induced Disseminated Intravascular Coagulation. » Crit Care Med. 2016;44(11):e1132-e1136).

Les échantillons de plasma (100 $\mu$L/puits) contenant les microparticules ont été ensuite déposés en double dans les puits et incubés pendant 30 minutes à 37°C. Après capture des MPs au fond des puits et lavage extensif, la réaction enzymatique prothrombinase a été réalisée. Les quantités de calcium et des facteurs de coagulation II, Va et Xa ajoutées ont été déterminées afin que la phosphatidylserine fixée sur les MPs soit le facteur limitant de la transformation de la thrombine en prothrombine. La quantité de thrombine générée a été révélée par son action lytique sur un substrat chromogénique (pNAPEP0216, 1,52mM, Cryopep, Montpellier, France). L'absorbance a été mesurée grâce à un spectrophotomètre (VersaMax Molecular Devices, Sunnylavale, Calfornie, USA) à la longueur d'onde de 405 nm. L'absorbance mesurée est proportionnelle à la quantité de PhtdSer exposée par les MPs. Les résultats sont exprimés en nanomolaires équivalents phosphatidylserine (nMeq. PhtdSer) par référence à une courbe de calibration réalisée avec des vésicules synthétiques aux quantités connues de PhtdSer.

**Statistique.**

Les variables ont été décrites comme des fréquences, et la comparaison a été faite par un test de $\chi^2$ ou un test exact de Fisher. Les données quantitatives ont été données en moyennes et déviations standards et analysées avec le test de Kruskall-Wallis. Les mesures répétées ont été analysées par ANOVA pour mesures répétées ou avec un modèle linéaire mixte. Les analyses post-hoc ont été réalisées en utilisant le test t avec correction de Bonferroni pour les comparaisons multiples. Avant de procéder à cette analyse, les variables ont été évaluées pour leur normalité et les variables dont la distribution n'est pas normale ont été transformées en utilisant soit une transformation logarithmique, racine carrée, inverse ou exponentielle. Plusieurs modèles de régression logistique ont été effectués pour expliquer la survenue d'une CIVD. Toutes les statistiques ont été réalisées avec le logiciel R version 3.1.3 software (R Core Team (2012). R: A language and environment for statistical computing. R Foundation for Statistical Computing, Vienna, Austria. http://www.Rproject.org/). Une valeur de $p<0,05$ a été considérée comme statistiquement significative.

**Principaux résultats:** 259 patients ont été analysés. 61 avaient une CIVD à l'admission (CIVD-J1) et 32 CIVD développées durant les 24 premières heures après l'admission (CIVD-J2). Plusieurs modèles de régression logistique ont confirmé que les MP dérivées des cellules endotheliales ont été associés à la CIVD: CD105 + -MPs (OR 2,13) et CD31 + -MPs (OR 0,65) (p <0,05). En outre, le rapport CD11a-microparticules sur leucocytes témoigne de l'activation des leucocytes (OR 1,59, p <0,05). La prédiction de la CIVD a également été analysée après exclusion des patients CIVD-J1. Une nouvelle analyse par régression logistique multiple a démontré l'association de CD105 -MPs (> 0,60 nM éq. PhtdSer, OR 1,67, p <0,01), numération plaquettaire ($\leq$127 G / L, OR 0,99, p <0,01) et le temps de prothrombine ($\leq$58%, OR 0,98, p <0,05) avec la CIVD. Un score combinant ces marqueurs à l'admission est prédictif de l'absence de CIVD (AUC 72,9%, une spécificité de 71,2%, une sensibilité de 71,0%, avec une valeur prédictive négative de 93,1% et une valeur prédictive positive de 31,0%).

**Résultats détaillés**

Afin d'analyser les patients lors de l'admission afin d'évaluer la prédiction de CIVD à J2 (CIVD-J2), nous avons exclus les patients CIVD-J1. Cent quatre-vingt-huit patients ont été analysés. La numération plaquettaire et le taux de prothrombine étaient significativement plus faibles chez les patients CIVD-J2 que chez les patients sans CIVD. Antithrombine et D-dimères n'étaient pas différents. A J2 et J3, la numération plaquettaire, le taux de prothrombine et l'antithrombine étaient inférieurs à J1 chez les patients CIVD-J2 et D-dimères étaient significativement plus élevés (p <0,05). Tous ces paramètres sont significativement différents chez les patients sans CIVD (p <0,05), avec une importante augmentation pour tous les trois paramètres. A noter, que les scores ISTH- « overt » et ISTH "non overt" ne permettent pas de prédire la CIVD.

**Les microparticules, marqueurs précoces de CIVD**

Les taux circulants de microparticules (MPs) totales sont élevés chez les patients en choc septique. L'activation cellulaire précède la CIVD: pour améliorer le diagnostic de la CIVD à J2, dès l'admission du patient de réanimation, nous avons analysé les microparticules endothéliales et l'activation des leucocytes.

CD105 + -MPs (p = 0,09) et CD11a + -Mps / leucocytes (p = 0,18) étaient associés à CIVD-J2 alors que ce n'est pas le cas des CD31 + -MPs.

L'analyse univariée réalisée sur les indicateurs biologiques de la CIVD évalués à J1, y compris des tests de routine et des microparticules, ont révélé quatre paramètres avec p <0,20: CD105 + -MPs, la numération plaquettaire, taux de prothrombine et ainsi que la fluorescence des neutrophiles (NEUT-FL). CD105-MPs, le taux de prothrombine et la numération plaquettaire ont été significativement associés à la CIVD dans une régression logistique multiple.

La performance individuelle de chaque paramètre est fortement augmentée par leur combinaison. L'équation de prédiction s'écrit:1,342 + (0,515 x [CD105$^+$ -MPs]) - (0,012 x [Plt]) - (0,018 x [PT]) avec [CD105$^+$-MPs] en nM éq. PtdSer, [Plt] en g / l et [PT] en pourcentage (%).

L'aire sous la courbe était de 72,9% avec IC à 95% [66,2 à 78,9] (p <0,001). Le seuil, Cut-off, était de -1,231 avec une spécificité de 71,2% [63,7 à 77,9], une sensibilité de 71,0% [52,0 à 85,8], une valeur prédictive négative de 93,1% [72,5 à 99,6] et une valeur prédictive positive de 31,0% [7,9 à 64,5].

Dans notre cohorte, 259 patients auraient été admissibles à un traitement anticoagulant spécifique établis dans des essais cliniques antérieurs. Selon JAAM, 61 avaient CIVD dès l'admission et peut-être immédiatement admissibles. Le calcul quotidien du score JAAM permettrait de diagnostiquer 31 autres patients avec CIVD éligibles pour un traitement anticoagulant parmi les 198 patients restants au jour 2. En utilisant notre score de prédiction, 125 patients pourraient être considérés à très faible risque de CIVD et auraient pu ne pas recevoir un traitement anticoagulant. Seulement 8 (6,5%) développeront une CIVD qui auraient été reconnue par JAAM au jour 2.

**Les microparticules, marqueurs précoces de CIVD**

Les taux circulants de microparticules (MPs) totales étaient élevés chez les patients en choc septique. Il n'y a pas de différence quantitative entre les MPs mesurées dans le groupe CIVD et dans le groupe non CIVD. En revanche, les patients présentant une CIVD présentaient une différence en termes de phénotype des microparticules plasmatiques. La quantité de MPs endothéliales CD105 était largement augmentée chez les patients avec CIVD par rapport à ceux n'ayant pas développé de CIVD (p<0,01). Cette différence existait dès J1 et persistait jusqu'à J7. Les MPs leucocytaires CD11a, reflétant l'activation leucocytaire, étaient significativement plus élevées chez les patients présentant une CIVD de J1 à J7. De la même façon, le rapport MPs leucocytaires CD11a/leucocytes était plus élevé chez les patients ayant développé une CIVD (p<0,05). Cette différence existait dès J1 et persistait (Figure 1C). De la même façon, il existait une augmentation significative des MPs neutrophiliques CD66b chez les patients avec CIVD. Le rapport MPs-CD66b/neutrophiles était augmenté chez les patients en choc septique et on a retrouvé la différence entre les deux groupes (p<0,01).

**Tableau 3.** Scores de CIVD à l'admission (J1)

| | Valeurs normales | Pas de CIVD (N=166) | "CIVDJ2" [a] (N=32) | "CIVD-J1" [b] (N=61) |
|---|---|---|---|---|
| **Temps au jour 2 (heures)** | | 16,9 [12,6-19,3] | 15,8 [12,9-18,7] | 16,7 [13,0-19,9] |
| **JAAM 2006** | | | | |
| J 1 | <4 | 1,8±0,7[#] | 2,4±0,6[$] | 5,8±1,6[#$] |
| J 2 | <4 | 1,6±0,9*[#] | 5,0±1,2* | 5,3±2,0[#] |
| **ISTH 2001 "overt"** | | | | |
| J 1 | <5 | 2,6±1,1[#] | 3,0±1,4[$] | 4,9±1,2[#$] |
| J 2 | <5 | 3,0±0,9*[#] | 4,6±0,9* | 5,0±1,3[#] |
| **ISTH 2001 "non overt"** | | | | |
| J 1 | <5 | 2,9±1,4*[#] | 3,8±1,3*[$] | 4,4±1,1[#$] |
| J 2 | <5 | 2,6±2,1*[#] | 5,0±1,4* | 3,7±2,3[#] |
| **SIRS score > 3** | | 166 (100,0) | 32 (100,0) | 61 (100,0) |
| **SOFA** | | 10,2±2,9*[#] | 12,3±2,4* | 13,5±2,8[#] |
| **SAPS II** | | 53,3±18,0*[#] | 62,4±19,9* | 63,5±17,1[#] |
| **Mortalité à J28** - N (%) | | 48 (28,3)*[#] | 13 (41,9)* | 29 (46,8)[#] |
| [a]" CIVD-J2": patients présentant les critères de CIVD à J2 et pas à J1; [b] "CIVDJ1": Patients présentant les critères de CIVD à J1 | | | | |
| *p<0,05 sans CIVD vs. CIVD-J2; [#]p<0. sans CIVD vs. CIVD-J1; [$]p<0,05 CIVD J1 vs. CIVDJ2 | | | | |

**Tableau 4.** Analyse par Régression logistique multiple de la valeur prédictive du score pour le diagnostique des patients CIVD J2 (patients CIVD J1 exclus)

| Paramètre | Odds-Ratio | 95% CI | *p* | Cut-off | Sensibilité (%) | Spécificité (%) |
|---|---|---|---|---|---|---|
| CD105+MPs | 1,67 | [1,20 -2,33] | 0,002 | >0,6 nM eqPS | 71,70 | 48,19 |
| Numération plaquettaire | 0,99 | [0,98- 0,99] | <0,00 1 | ≤127 G/L | 63,64 | 90,00 |
| Taux de Prothrombin | 0,98 | [0,97-1,00] | 0,038 | ≤58 % | 81,82 | 60,00 |
| CD11a+-MPs/Leucocytes and antithrombine n'ont pas été retenus (p>0,25) | | | | | | |

**Exemple 2 Exemple d'un score combinant la fluorescence des neutrophiles, le taux de prothrombine, la numération plaquettaire et les microparticules CD105-MP Patients**

150 patients consécutifs d'âge adulte, remplissant tous les critères de choc septique à l'admission aux soins intensifs, ont été inclus dans l'étude. Les patients atteints de maladies chroniques à un stade terminal ainsi que les patients souffrant de neutropénie ont été exclus de l'étude Selon le score JAAM, 35% des patients présentent une CIVD. Les patients présentant une CIVD précoce induite par le choc septique étaient plus gravement malades avec des scores de gravité SOFA (Sequential Organ Failure Assesment Score) et SAPS2 (Simplified Acute Physiology Score) ainsi qu'une mortalité plus élevée statistiquement dans le groupe CIVD (tableau 5). Les numérations des leucocytes et des neutrophiles étaient significativement inférieures chez les patients atteints de CIVD tandis la quantification de l'hémoglobine et du sodium n'est pas altérée, excluant de fait un effet de dilution du plasma.

**Prélèvements sanguins :**

En sus des prélèvements présentés dans l'exemple 1, des tubes ont été prélevés pour les analyses nécessaires aux soins des patients, dont un tube EDTA (Vacutainer™, Becton Dickinson, Le Pont de Claix, France) et utilisés pour la détection de la fluorescence liée à la décompaction de l'ADN. Au cours du processus de validation, nous avons analysé, depuis des échantillons prélevés sur des volontaires sains, l'activation ex- vivo des neutrophiles stimulés par une concentration de 4 $\mu$M d'ionomycine (Calbiochem, Merck Biodeveloppement, Martillac, France)

**Numérations et formules sanguines**

L'intensité du marquage par l'intercalant de l'ADN qui témoigne de l'accessibilité du double brin (cellule immature, activation cellulaire...), a été identifiée comme NEUT-FL. La norme du paramètre NEUT-FL a été établie à partir de 1300 bilans, soit à partir de l'ensemble des patients majeurs ayant eu un bilan biologique pendant une période de 24 heures au CHU de STRASBOURG sur les deux sites hospitaliers regroupant l'ensemble des spécialités médicales et chirurgicales adultes.

**Analyse de la morphologie des cellules**

Des frottis sanguins ont été effectués sur les patients et échantillonnés en sur tube EDTA (7,2mg/4mL) / citrate (0,129M) puis soumis à une coloration de May-Grünwald-Giemsa. L'échantillonnage des volontaires sains a quant à lui été effectué depuis un prélèvement sanguin veineux périphérique.

Statistique :

Les variables catégorielles (ou qualitatives) ont été analyses par le test exact de Fisher et les variables continues par le test des rangs signés de Wilcoxon. La normalité de la distribution a été analysée par le test de Shapiro-Wilk. La méthode de Bonferroni-Holm a été utilisée pour obtenir les « p-values » ajustées pour les comparaisons multiples. L'analyse des aires sous les courbes ROC (Receiver-operating characteristic) a été réalisée et le coefficient de corrélation de Spearman déterminé. On considère statistiquement significatif une « p-value » inférieure à 0,05. Toutes les analyses statistiques ont été effectuées à l'aide du logiciel R version 3.1.3 software (R Core Team (2012). R: A language and environment for statistical computing. R Foundation for Statistical Computing, Vienna, Austria. http://www.Rproject.org/). Les variables asymétriques sont présentés sous la forme de valeurs médianes avec interquartiles grâce au logiciel GraphPad Prism® version 6 (La Jolla, CA, USA).

**Résultats** :

**Numérations leucocytaires** Les numérations leucocytaires et des neutrophiles étaient significativement plus basses chez les patients présentant une CIVD. La fluorescence des neutrophiles était augmentée chez l'ensemble des patients en choc septique (p<0,01).

**Cyto-morphologie des neutrophiles**

On a observé, chez les patients étudiés, que l'on peut associer la CIVD avec une altération de la morphologie des neutrophiles observés depuis les frottis sanguins par la coloration de May-Grünwald-Giemsa comme le montre la figure 5 D. En effet, on observe notamment une diminution dans la granularité et la lobulation du noyau des Neutrophiles. Ces altérations peuvent être quantifiées par un score cytologique visant l'obtention d'une corrélation positive et significative (p<0,05) entre sa valeur et la CIVD durant les 3 premiers jours d'échantillonnage.

**Fluorescence des leucocytes**

La fluorescence moyenne des polynucléaires neutrophiles (PNN) mesurée en unités arbitraires (U.A.) était significativement augmentée chez les patients présentant une CIVD précoce définie par un score JAAM $\geq$ 4 à J1 ou J2 par rapport au groupe absence de CIVD et la différence est significative (p<0,01) : 70,0 [66,4-81,8] *vs.* 50,7 [46,3-53,8] UA, p<0,05, les valeurs normales établis dans notre laboratoire à partir de volontaires sains étant : 44,8 [43,5-46,9] UA, *p*<0,05 et les valeurs moyenne mesurées chez 1340 patients adultes du CHU soit l'ensemble des patients ayant eu une numération sanguine sur une période de 24 heures étant : 46,80 [42,7-50,4] UA. Une courbe ROC a été établie : l'aire sous la courbe était de 0,882 (p<0,0001) pour le diagnostic de CIVD avec un seuil (cut-off) à 57,3 UA au-delà duquel l'augmentation de la fluorescence est prédictive d'une CIVD avec une sensibilité de 90,9% et une spécificité de 80,6%.

L'analyse univariée réalisée sur les indicateurs biologiques de la CIVD évalués à J1, y compris des tests de routine et des microparticules, ont révélé quatre paramètres avec p <0,20: CD105 [+]-MPs, la numération plaquettaire, taux de prothrombine et ainsi que la fluorescence des neutrophiles (NEUT-FL).

**Tableau 5.** Caractéristiques des patients

|  | CIVD | Pas de CIVD | p |
|---|---|---|---|
| SAPS 2 | 60,4±15,2 | 52,7±18,0 | 0,01 |
| SOFA | 12,5±2,6 | 9,9±3,2 | 0,01 |
| Mortalité au jour 7 - N (%) | 20,0 | 5,5 | 0,01 |
| Noradrénaline - N (%) | 35 (100,0) | 65 (100,0) | 1,00 |
| Dobutamine - N (%) | 7 (20,0) | 13 (17,8) | 1,00 |
| Ventilation mécanique - N (%) | 33 (94,3) | 61 (93,8) | 1,00 |
| Epuration extra-rénale - N (%) | 16 (45,7) | 19 (29,2) | 0,13 |
| JAAM 2006 | 4,6±2,0 | 1,9±0,7 | 0,01 |
| ISTH 2001 "overt" | 4,4±1,2 | 2,9±1,0 | 0,01 |
| ISTH 2001 "non overt" | 4,6±1,3 | 3,0±1,3 | 0,05 |
| Taux de prothrombine (%) | 44 [33-62] | 64 [51-81] | 0,01 |
| D-dimères (μg/L) | 7,63 [3,01-20,00] | 3,31 [1,89-5,44] | 0,01 |
| Fibrinogène (g/L) | 5,58 [3,52-9,56] | 6,40 [4,67-7,68] | 0,03 |
| Leucocytes (G/L) | 10,3 [3,6-22,8] | 14,3 [11,6-20,1] | 0,05 |
| Numération des Neutrophiles (G/L) | 8,3 [4,0-19,9] | 14,0 [10,0-19,8] | 0,01 |
| Fluorescence des neutrophiles (AU) | 66,6 [59,3-80,4] | 50,0 [46,6-56,2] | 0,01 |
| Numération des Plaquettes (G/L) | 80 [45-132] | 179 [140-190] | 0,05 |
| Hémoglobine (g/dL) | 10,5 [9,1-12,4] | 10,3 [9,6-12,1] | 0,35 |

(suite)

|  | CIVD | Pas de CIVD | *p* |
|---|---|---|---|
| Protéines (g/L) | 52 [40-56] | 56 [48-64] | 0,43 |
| Sodium (mmol/L) | 136 [133-138] | 136 [132-140] | 1,00 |
| Protéine C-Réactive (CRP) (mg/L) | 214 [84-282] | 173 [65-278] | 0,29 |

L'analyse multivariée réalisée sur les paramètres biologiques mesurés à J1 et éligibles (p<0,20) lors de l'analyse univariée ci-dessus figure dans le tableau 6.

**Tableau 6** : Analyse multivariée

| Paramètres | odd ratio | Intervalle de confiance à 95% | p |
|---|---|---|---|
| Fluorescence des neutrophiles > 57 UA | 2,829 | 1,185 à 7,086 | 0,022 |
| CD105 $^+$-MPs > 0,65 nM éq. PS | 2,343 | 1,289 à 5,236 | 0,017 |
| Numération des Plaquettes < 127 G/L | 0,990 | 0,983 à 0,995 | <0,001 |
| Taux de Prothrombine < 58,5% | 0,983 | 0,964 à 0,001 | 0,070 |

L'application du procédé de calcul du score de prédiction $S_1$ à partir de la formule suivante :

$$S_1 = 0{,}786 + 1{,}040 * V_{neutfl} + 0{,}851 * V_{CD105} + (-0{,}010 * V_{Plq}) + (-0{,}017 * V_{TP})$$

Dans laquelle la valeur Vneutfl était égale à 1 si la fluorescence des neutrophiles dans un échantillon de plasma était supérieure à 57UA, égale à 0 si la fluorescence des neutrophiles était inférieure ou égale à 57UA, une valeur $V_{CD105}$ égale à 1 si la concentration CD105 $^+$-MPs était supérieure à 0,65 nM éq. PS ou d'une valeur $V_{CD105}$ égale à 0 si la concentration CD105 $^+$-MPs était est inférieure à 0,65 nM éq. PS, une valeur $V_{Plq}$ égale à 1 si la concentration plaquettaire était inférieur à 127G/L ou d'une valeur $V_{Plq}$ égale à 0 si la concentration plaquettaire était est supérieure 127G/L, d'une valeur $V_{TP}$ égale à 1 si le taux de prothrombine était inférieur à 58,5% ou d'une valeur $V_{TP}$ égale à 0 si le taux de prothrombine était est supérieur à 58,5%.

Une valeur de S supérieure à -0,47 indiquant une CIVD et/ou l'apparition d'une CIVD.

Le procédé a été également mis en œuvre avec le score de prédiction suivant :

$$S_1 = \varepsilon + V_{neutfl} \times \alpha + V_{CD105} \times \beta + V_{plaquettes} \times \Omega + V_{TP} \times \theta$$

dans laquelle $\varepsilon$ est compris de -0,708 à 2,280, $\alpha$ est compris de 0,152 à 1,928, $\beta$ est compris entre 0,154 à 1,549, $\Omega$ est compris entre -0,016 à - 0,005, et $\theta$ est compris entre -0,036 à 0,001,

Ce score a été réalisé sur la base d'un intervalle de confiance à 95% des différents coefficients et permet de déterminer la présence et/ou l'apparition d'une CIVD.

**Exemple 3 Exemple d'un score combinant la fluorescence des neutrophiles, le taux de prothrombine et la numération plaquettaire**

Dans cet exemple, la détermination de l'algorithme de détection/prédiction de la CIVD a été effectuée à partir de données cliniques obtenues à l'exemple 2 précité.

La construction de l'algorithme a été menée à l'aide de la modélisation par régression logistique de la CIVD précoce (Pré CIVD) dans la cohorte de patients de l'exemple 2 : analyse univariée puis multivariée pour les variables liées significativement à la survenue d'une CIVD précoce avec p≤0,20.

Dans ce contexte, chaque patient a été caractérisé par les valeurs de mesure de la fluorescence des neutrophiles, du taux de prothrombine et de la numération plaquettaire calculées suivant le procédé décrit dans l'exemple 2.

L'application du procédé de calcul du score de prédiction $S_2$ à partir de la formule suivante :

$$S_2 = 1{,}726 + 1{,}060 * V_{neutfl} + (-0{,}010 * V_{Plq}) + (-0{,}022 * V_{TP})$$

Dans laquelle la valeur $V_{neutfl}$ était égale à 1 si la fluorescence des neutrophiles dans un échantillon de plasma était supérieure à 57UA, égale à 0 si la fluorescence des neutrophiles était inférieure ou égale à 57UA, une valeur $V_{Plq}$ égale à 1 si la concentration plaquettaire était inférieur à 127G/L ou d'une valeur $V_{Plq}$ égale à 0 si la concentration plaquettaire était est supérieure 127G/L, d'une valeur $V_{TP}$ égale à 1 si le taux de prothrombine était inférieur à 58,5% ou d'une valeur $V_{TP}$ égale à 0 si le taux de prothrombine était est supérieur à 58,5%.

Une valeur de S supérieure à 0,16 indiquant une CIVD et/ou l'apparition d'une CIVD.

Le procédé a été également mis en œuvre avec le score $S_2$ de prédiction suivant :

$$S_2 = \varepsilon + V_{neutfl} \times \alpha + V_{Plq} \times \Omega + V_{TP} \times \theta$$

dans laquelle $\varepsilon$ est compris de 0,381 à 3,070, $\alpha$ est compris de 0,209 à 1,910, $\Omega$ est compris entre -0,015 à -0,005, et $\theta$ est compris entre -0,040 à -0,004.

Ce score a été réalisé sur la base d'un intervalle de confiance à 95% des différents coefficients et permet de déterminer la présence et/ou l'apparition d'une CIVD.

**Exemple 4. Identification de marqueurs de NETs associés aux neutrophiles fluorescents à l'aide de la cytométrie en flux. Résumé :**

L'identification de neutrophiles fluorescents a été réalisée par cytométrie en flux conventionnelle en utilisant une méthode de marquage proche de celle décrite dans l'exemple 2 ci-dessus. Les neutrophiles sanguins ont été identifiés à l'aide d'une combinaison d'anticorps fluorescents anti leucocytes (marqueur pan leucocytaire CD45), anti polynucléaires éosinophiles (CD49d), et monocytes (CD14). Le marquage à l'aide d'intercalant nucléique a été réalisé après lyse érythrocytaire. L'acquisition dans les 5 minutes suivant la procédure de marquage a permis de révéler une population neutrophilique de fluorescence supérieure dans les échantillons sanguins de patients avec CIVD comparativement aux patients sans sepsis. (Figure 6A)

**Objectifs** :

Confronter la présence d'une population de neutrophiles à fluorescence importante à la présence de NETs signant la Netose pouvant par exemple être associée à la CIVD. Des échantillons sanguins de sujets exempts de sepsis et dans lesquels la Netose a été provoquée par l'ajout de ionomycine (4 $\mu$M, 30 minutes à 37 °C), ou de patient septique et évalué deux marqueurs de Nets, respectivement les histones H3 citrullinées et l'activité myeloperoxydase par cytométrie en flux conventionnelle ont été étudié. Les marquages extracellulaires signant la présence d'antigène accessibles à la surface de surface cellulaire et intracellulaire, après perméabilisation, ont été réalisés.

Le matériel et méthode est détaillé ci-dessous :

Prélèvements sanguins: Ils ont été réalisés dans des tubes utilisant l'héparinate de calcium comme anti-coa. La stimulation par ionomycine (4 $\mu$M) des échantillons de sujets sans sepsis et avec numération formule sanguine normale a été réalisée pendant 30 minutes à 37°C et à concentration calcique finale 2 mM. Pour ce faire 3,2$\mu$l de solution de ionomycine a été introduite dans 50$\mu$l d'échantillon de sang La ionomycine a été ensuite éliminée par centrifugation à basse vitesse à savoir 540G pendant 5 minutes.

Identification des neutrophiles à forte fluorescence: Les échantillons sanguins ont été dilués dans un tampon phosphate buffer saline (PBS) 1X avant incubation 5 minutes avec une combinaison d'anticorps fluorescents pan leucocytaires (CD45-FITC), anti polynucléaires éosinophiles (CD49d-PE), et monocytes anti-CD14-APC de la marque Biocytex, suivie d'une lyse érythrocytaire (slow lyse, Biocytex). Un intercalant nucléique (SYTO 40, 1,33 mM ou SYTO RNA Select 133,5 $\mu$M Thermofisher) a été ensuite appliqué 5 minutes avant acquisition de l'échantillon dans un cytomètre conventionnel (FacsCalibur, Becton Dickinson).

Identification des Nets associés aux neutrophiles : Les leucocytes stimulés ou non par la ionomycine ont été marqués par incubation 20 min à température ambiante avec une combinaison d'anticorps : pan leucocytaire anti-CD45 clone H130 (V500, clone H130, BD biosciences), anti neutrophile anti-CD16 clone 3G8 (BV421, BD biosciences), anti-MPO (clone 5B8, BD Biosciences) et anti-histone citrullinée (anticorps polyclonal ab5103 Abcam). Pour ce faire, 50$\mu$l de sang ont été incubés avec les anticorps décrits ci-dessus. Le marquage intracellulaire a été réalisé après perméabilisation (BD Facs Permeabilising solution diluée au dixième, BD biosciences). Tous les anticorps ont été incubés 20 minutes à température ambiante, à savoir 20°C à l'abri de la lumière. Après lavage en tampon PBS contenant 0,5 % de SVF (Sérum de Vœu fœtal), un anticorps secondaire anti-IgG de lapin conjugué à Alexa 700 (A21038, Thermofischer) a été incubé pendant 20 minutes à température ambiante à savoir 20°C à l'abri de la lumière pour compléter le marquage secondaire des immunoglobulines anti histones. L'acquisition des échantillons dans un cytomètre conventionnel (Becton Dickinson) a été réalisée après lavage en tampon PBS contenant 0,5 % de SVF.

Principaux résultats: tel que représenté sur la figure 6A, en présence de CIVD, la fluorescence moyenne des neutrophiles exprimée en unités arbitraires était significativement supérieure à celle des sujets indemnes de sepsis en utilisant la cytométrie conventionnelle (p<0,01). Les marquages extracellulaires et intracellulaires par anticorps anti histone citrullinée (Figure 6C I et II respectivement) et anti myeloperoxydase (Figure 6 B I et II respectivement) ont révélé que (i) la Netose se traduit par l'apparition de neutrophiles de taille supérieure avec (ii) une moyenne de fluorescence augmentée après marquage extracellulaire par les anticorps anti-histones citrullinées tandis que le marquage intracellulaire diminue (iii) symétriquement, la moyenne de fluorescence après marquage extracellulaire par anticorps anti myeloperoxydase était supérieure dans la population neutrophilique de grande taille tandis que la moyenne de fluorescence intracellulaire diminue.

Ces mesures démontrent clairement que la cytométrie en flux conventionnelle permet d'identifier une fluorescence neutrophilique augmentée dans un échantillon de patient CIVD. En outre, cet exemple démontre clairement qu'une cytométrie en flux conventionnelle permet d'identifier une fluorescence des neutrophiles, par exemple à l'aide de sondes intercalentes d'acides nucléiques et également la présence de Netose, par exemple déclenchée par un agent exogène (ionomycine) ou dans un échantillon sanguin de patient CIVD par marquage des histones citrullinées et de la myeloperoxydase de la population neutrophilique.

## Liste de Références

1. Fourrier F. Severe sepsis, coagulation, and fibrinolysis: dead end or one way? Crit Care Med 2012;40(9):2704-2708.

2. Gando S, Iba T, Eguchi Y, et al. A multicenter, prospective validation of disseminated intravascular coagulation diagnostic criteria for critically ill patients: comparing current criteria. Crit Care Med 2006;34(3):625-631.

3. Taylor FB, Jr., Toh CH, Hoots WK, et al. Towards définition, clinical and laboratory criteria, and a scoring system for disseminated intravascular coagulation. Thromb Haemost 2001;86(5):1327-1330.

4. Delabranche X, Boisrame-Helms J, Asfar P, et al. Microparticles are new biomarkers of septic shock-induced disseminated intravascular coagulopathy. Intensive Care Med 2013;39(10):1695-1703.

5. Chung S, Kim JE, Park S, et al. Neutrophil and monocyte activation markers have prognostic impact in disseminated intravascular coagulation: in vitro effect of thrombin on monocyte CD163 shedding. Thromb Res 2011;127(5):450-456.

6. Engelmann B, Massberg S. Thrombosis as an intravascular effector of innate immunity. Nature reviews Immunology 2013;13(1):34-45.

7. Zonneveld R, Molema G, Plotz FB. Analyzing Neutrophil Morphology, Mechanics, and Motility in Sepsis: Options and Challenges for Novel Bedside Technologies. Crit Care Med 2015.

8. Matsumoto H. The technology of reagents in the automated hematology analyzers Sysmex XE-2100™ - Red fluorescence technology. Sysmex J Int 1999;9:179-185.

9. Park SH, Park CJ, Lee BR, et al. Sepsis affects most routine and cell population data (CPD) obtained using the Sysmex XN-2000 blood cell analyzer: neutrophil-related CPD NE-SFL and NE-WY provide useful information for detecting sepsis. International journal of laboratory hematology 2015;37(2):190-198.

10. Angus DC, van der Poll T. Severe sepsis and septic shock. N Engl J Med 2013; 369: 840-851.

11. Van der Poll T, Herwald H. The coagulation system and its function in early immune defense. Thromb Haemost. 2014; 112: 640-8.

12. Engelmann B, Massberg S. Thrombosis as an intravascular effector of innate immunity. Nat Rev Immunol. 2013; 13: 34-45.

13. Wada H, Matsumoto T, Yamashita Y. Diagnosis and treatment of disseminated intravascular coagulation (DIC) according to four DIC guidelines. J Intensive Care. 2014; 2: 15.

14. Gando S, Wada H, Thachil J, Scientific, Standardization Committee on DIC of the International Society on Thrombosis and Haemostasis. Differentiating disseminated intravascular coagulation (DIC) with the fibrinolytic phenotype from coagulopathy of trauma and acute coagulopathy of trauma-shock (COT/ACOTS). J Thromb Haemost. 2013; 11: 826-35.

15. Gando S, Otomo Y. Local hemostasis, immunothrombosis, and systemic disseminated intravascular coagulation in trauma and traumatic shock. Crit Care. 2015; 19: 72.

16. Fourrier F. Severe sepsis, coagulation, and fibrinolysis: dead end or one way? Crit Care Med. 2012; 40: 2704-8.

17. Levi M. The coagulant response in sepsis and inflammation. Hamostaseologie. 2010; 30: 10-2, 4-6.

18. Levi M, van der Poll T. Endothelial injury in sepsis. Intensive Care Med. 2013; 39: 1839-42.

19. Marshall JC. Why have clinical trials in sepsis failed? Trends Mol Med. 2014; 20: 195-203.

20. Meziani F, Delabranche X, Asfar P, et al. Bench-to-bedside review: circulating microparticles--a new player in sepsis? Crit Care. 2010; 14: 236.

21. Delabranche X, Boisrame-Helms J, Asfar P, et al. Microparticles are new biomarkers of septic shock-induced disseminated intravascular coagulopathy. Intensive Care Med. 2013; 39: 1695-703.

22. Levy MM, Fink MP, Marshall JC, et al. 2001 SCCM/ESICM/ACCP/ATS/SIS International Sepsis Definitions Conference. Intensive Care Med. 2003; 29: 530-8.

23. Taylor FB, Jr., Toh CH, Hoots WK, et al. Towards définition, clinical and laboratory criteria, and a scoring system for disseminated intravascular coagulation. Thromb Haemost. 2001; 86: 1327-30.

24. Gando S, Iba T, Eguchi Y, et al. A multicenter, prospective validation of disseminated intravascular coagulation diagnostic criteria for critically ill patients: comparing current criteria. Crit Care Med. 2006; 34: 625-31.

25. Hugel B, Zobairi F, Freyssinet JM. Measuring circulating cell-derived microparticles. J Thromb Haemost. 2004; 2: 1846-7. 22

26. Spero JA, Lewis JH, Hasiba U. Disseminated intravascular coagulation. Findings in 346 patients. Thromb Haemost 1980; 43: 28-33.

27. Dellinger RP, Levy MM, Rhodes A, et al. Surviving sepsis campaign: international guidelines for management of severe sepsis and septic shock: 2012. Crit Care Med. 2013; 41: 580-637.

28. Tagami T, Matsui H, Horiguchi H, et al. Antithrombin and mortality in severe pneumonia patients with sepsis-associated disseminated intravascular coagulation: an observational nationwide study. J Thromb Haemost. 2014; 12: 1470-9.

29. Gando S, Saitoh D, Ogura H, et al. A multicenter, prospective validation study of the Japanese Association for Acute Medicine disseminated intravascular coagulation scoring system in patients with severe sepsis. Crit Care. 2013; 17: R111.

30. de Stoppelaar SF, van 't Veer C, van der Poll T. The role of platelets in sepsis. Thromb Haemost. 2014; 112: 666-77.

31. Hamzeh-Cognasse H, Damien P, Chabert A, et al. Platelets and infections - complex interactions with bacteria. Front Immunol. 2015; 6: 82.

32. Aupeix K, Hugel B, Martin T, et al. The significance of shed membrane particles during programmed cell death in vitro, and in vivo, in HIV-1 infection. J Clin Invest. 1997; 99: 1546-54.

33. Lacroix R, Robert S, Poncelet P, et al. Overcoming limitations of microparticle measurement by flow cytometry. Semin Thromb Hemost. 2010; 36: 807-18.

34. Robert S, Lacroix R, Poncelet P, et al. High-sensitivity flow cytometry provides access to standardized measurement of small-size microparticles--brief report. Arterioscler Thromb Vasc Biol. 2012; 32: 1054-8.

35. Lacroix R, Judicone C, Mooberry M, Boucekine M, Key NS, Dignat-George F, The ISSCW. Standardization of pre-analytical variables in plasma microparticle determination: results of the International Society on Thrombosis and Haemostasis SSC Collaborative workshop. J Thromb Haemost. 2013; 11: 1190-3.

36. Brenner T, Schmidt K, Delang M, et al. Viscoelastic and aggregometric point-of-care testing in patients with septic shock - cross-links between inflammation and haemostasis. Acta Anaesthesiol Scand 2012; 56: 1277-90.

37. Panigada M, Zacchetti L, L'Acqua C, et al. Assessment of fibrinolysis in sepsis patients with urokinase modified thromboelastography. PLoS One 2015; 10: e0136463.

38. Haase N, Ostrowski SR, Wetterslev J, et al. Thromboelastography in patients with severe sepsis: a prospective cohort study. Intensive Care Med 2015; 41: 77-85. 23

## Revendications

1. Procédé in-vitro de prédiction et/ou de détection de la coagulation intravasculaire disséminée (CIVD) à partir d'un premier échantillon biologique comprenant une mesure de la fluorescence de neutrophiles avec un fluorochrome intercalant de l'ADN.

2. Procédé de détection selon la revendication 1, comprenant en outre une étape de comparaison de la valeur mesurée avec une valeur de référence.

3. Procédé de de détection selon la revendication 1 ou 2, dans lequel l'échantillon biologique est choisi dans le groupe comprenant un échantillon de sang.

4. Procédé selon l'une quelconque des revendications 1 à 3, comprenant en outre une étape de mesure à partir d'un second échantillon biologique de la concentration $RM_{CD105}$ de microparticules membranaires porteuses du le cluster de différenciation 105 (CD105).

**5.** Procédé selon l'une quelconque des revendications 1 à 4, dans lequel lesdits premier et second échantillons biologiques sont un seul et même échantillon.

**6.** Procédé selon l'une quelconque des revendications 1 à 5, dans lequel lesdits premier et second échantillons biologiques proviennent d'un patient présentant un choc septique.

**7.** Procédé selon l'une quelconque des revendications 1 à 6, dans lequel la valeur de référence fluorescence de neutrophiles est supérieure à 57 UA.

**8.** Procédé selon l'une quelconque des revendications 4 à 7, dans lequel la valeur de référence $R_{CD105}$ est supérieure à 0,65 nM eq PS.

**9.** Procédé de détection et/ou de suivi de l'évolution d'une coagulation intravasculaire disséminée (CIVD) à partir d'un échantillon biologique comprenant les étapes suivantes :

    a. mesure de la fluorescence des neutrophiles $R_{0neutfl}$ avec un fluorochrome intercalant de l'ADN
    b. comparaison de la valeur mesurée de fluorescence des neutrophiles ROneutfl avec une valeur de référence RNeutfl
    c. mesure de la concentration $R_{0CD105}$ de microparticules membranaire comprenant le cluster de différenciation 105 (CD105),
    d. comparaison de la valeur mesurée $R_{0CD105}$ avec une valeur de référence $R_{CD105}$
    e. mesure de la concentration plaquettaire RoPlt
    f. comparaison de la valeur mesurée RoPlt avec une valeur de référence RPlt
    g. mesure du taux de prothrombine RoTP
    h. comparaison de la valeur mesurée RoTP avec une valeur de référence RTP
    i. affectation d'une valeur fluorescence des neutrophiles Vneutfl égale à 1 si ROneutfl est supérieure à la valeur $R_{neutfl}$ ou d'une valeur Vneutfl égale à 0 si ROneutfl est inférieure à la valeur $R_{neutfl}$
    j. affectation d'une valeur $V_{CD105}$ égale à 1 si $R_{0CD105}$ est supérieur à la valeur $R_{CD105}$ ou d'une valeur CD 105 égale à 0 si $R_{0CD105}$ est inférieur à la valeur $R_{CD105}$
    k. affectation d'une valeur $V_{Plq}$ égale à 1 si RoPlt est inférieure à la valeur RPlt ou d'une valeur $V_{Plq}$ égale à 0 si RoPlt est supérieure à la valeur RPlt
    l. affectation d'une valeur $V_{TP}$ égale à 1 si RoTP est inférieure à la valeur RTP ou d'une valeur $V_{TP}$ égale à 0 si RoTP est supérieure à la valeur RTP
    m. calcul du score S1 selon la formule suivante :

$$S_1 = \varepsilon + V_{neutfl} \times \alpha + V_{CD105} \times \beta + V_{Plq} \times \Omega + V_{TP} \times \theta$$

    dans laquelle $\varepsilon$ est compris de -0,708 à 2,280, $\alpha$ est compris de 0,152 à 1,928, $\beta$ est compris entre 0,154 à 1,549, $\Omega$ est compris entre -0,016 à -0,005, et $\theta$ est compris entre -0,036 à 0,001,
    ou

$$S1 = 0,786 + 1,040 \times V_{neutfl} + 0,851 \times V_{CD105} + (-0,010 \times V_{Plq}) + (-0,017 \times V_{TP})$$

**10.** Procédé de détection et/ou de suivi de l'évolution d'une coagulation intravasculaire disséminée (CIVD) à partir d'un échantillon biologique comprenant les étapes suivantes :

    a. mesure de la fluorescence des neutrophiles $R_{0neutfl}$ avec un fluorochrome intercalant de l'ADN
    b. comparaison de la valeur mesurée de fluorescence des neutrophiles ROneutfl avec une valeur de référence RNeutfl
    c. mesure de la concentration plaquettaire RoPlt
    d. comparaison de la valeur mesurée RoPlt avec une valeur de référence RPlt
    e. mesure du taux de prothrombine RoTP
    f. comparaison de la valeur mesurée RoTP avec une valeur de référence RTP

g. affectation d'une valeur fluorescence des neutrophiles Vneutfl égale à 1 si ROneutfl est supérieure à la valeur $R_{neutfl}$ ou d'une valeur Vneutfl égale à 0 si ROneutfl est inférieure à la valeur $R_{neutsfl}$

h. affectation d'une valeur $V_{Plq}$ égale à 1 si RoPlt est inférieure à la valeur RPlt ou d'une valeur $V_{Plq}$ égale à 0 si RoPlt est supérieure à la valeur RPlt

i. affectation d'une valeur $V_{TP}$ égale à 1 si RoTP est inférieure à la valeur RTP ou d'une valeur $V_{TP}$ égale à 0 si RoTP est supérieure à la valeur RTP

j. calcul du score S2 selon la formule suivante :

$$S2 = \varepsilon + \text{Vneutfl} \times \alpha + V_{plaquettes} \times \Omega + V_{TP} \times \theta$$

dans laquelle $\varepsilon$ est compris de 0,381 à 3,070, $\alpha$ est compris de 0,209 à 1,910, $\Omega$ est compris entre -0,015 à -0,005, $\theta$ est compris entre -0,040 à -0,004

ou

$$S2 = 1,726 + 1,060*V_{neutfl} + (-0,010*V_{Plq}) + (-0,022*V_{TP}),$$

**11.** Procédé de détection et/ou de suivi de l'évolution d'une coagulation intravasculaire disséminée (CIVD) à partir d'un échantillon biologique comprenant les étapes suivantes :

a. mesure de la fluorescence des neutrophiles $R_{0neutfl}$ avec un fluorochrome intercalant de l'ADN

b. comparaison de la valeur mesurée de fluorescence des neutrophiles ROneutfl avec une valeur de référence RNeutfl

c. mesure de la concentration $R_{0CD105}$ de microparticules membranaire comprenant le cluster de différenciation 105 (CD105),

d. comparaison de la valeur mesurée $R_{0CD105}$ avec une valeur de référence $R_{CD105}$

e. mesure de la concentration plaquettaire RoPlt

f. comparaison de la valeur mesurée RoPlt avec une valeur de référence RPlt

g. mesure du taux de prothrombine RoTP

h. comparaison de la valeur mesurée RoTP avec une valeur de référence RTP

i. affectation d'une valeur fluorescence des neutrophiles Vneutfl égale à 1 si ROneutfl est supérieure à la valeur $R_{neutsfl}$ ou d'une valeur Vneutfl égale à 0 si ROneutfl est inférieure à la valeur $R_{neutfl}$

j. affectation d'une valeur $V_{CD105}$ égale à 1 si $R_{0CD105}$ est supérieur à la valeur $R_{CD105}$ ou d'une valeur CD 105 égale à 0 si $R_{0CD105}$ est inférieur à la valeur $R_{CD105}$

k. affectation d'une valeur $V_{Plq}$ égale à 1 si RoPlt est supérieur à la valeur RPlt ou d'une valeur $V_{Plq}$ égale à 0 si RoPlt est inférieur à la valeur RPlt

l. affectation d'une valeur $V_{TP}$ égale à 1 si RoTP est supérieur à la valeur RTP ou d'une valeur $V_{TP}$ égale à 0 si RoTP est inférieur à la valeur RTP

n. calcul du score S1 et/ou S2 selon la formule suivante :

$$S_1 = \varepsilon + V_{neutfl} \times \alpha + V_{CD105} \times \beta + V_{Plq} \times \Omega + V_{TP} \times \theta$$

dans laquelle $\varepsilon$ est compris de -0,708 à 2,280, $\alpha$ est compris de 0,152 à 1,928, $\beta$ est compris entre 0,154 à 1,549, $\Omega$ est compris entre -0,016 à -0,005, et $\theta$ est compris entre -0,036 à 0,001,

$$S_2 = \varepsilon + V_{neutfl} \times \alpha + V_{Plq} \times \Omega + V_{TP} \times \theta$$

dans laquelle $\varepsilon$ est compris de 0,381 à 3,070, $\alpha$ est compris de 0,209 à 1,910, $\Omega$ est compris entre -0,015 à -0,005, $\theta$ est compris entre -0,040 à -0,004.

**Patentansprüche**

**1.** In-vitro-Verfahren zum Vorhersagen und/oder Nachweisen von disseminierter intravasaler Koagulopathie (DIC) ausgehend von einer ersten biologischen Probe, das eine Messung der Neutrophilenfluoreszenz mit einem DNA-interkalierenden Fluorochrom umfasst.

2. Nachweisverfahren nach Anspruch 1, das unter anderem einen Schritt des Vergleichens des gemessenen Werts mit einem Referenzwert umfasst.

3. Nachweisverfahren nach Anspruch 1 oder 2, wobei die biologische Probe ausgewählt ist aus der eine Blutprobe umfassenden Gruppe.

4. Verfahren nach einem der Ansprüche 1 bis 3, ferner umfassend einen Schritt des Messens, ausgehend von einer zweiten biologischen Probe, der $Rm_{CD105}$-Konzentration von Membranmikropartikeln, die den "Cluster der Differenzierung" 105 (CD105) tragen.

5. Verfahren nach einem der Ansprüche 1 bis 4, wobei die erste und die zweite biologische Probe ein und dieselbe Probe sind.

6. Verfahren nach einem der Ansprüche 1 bis 5, wobei die erste und die zweite biologische Probe von einem Patienten mit septischem Schock stammen.

7. Verfahren nach einem der Ansprüche 1 bis 6, wobei der Neutrophilenfluoreszenz-Referenzwert größer als 57 AU ist.

8. Verfahren nach einem der Ansprüche 4 bis 7, wobei der Referenzwert $R_{CD105}$ größer als 0,65 nM Äq. PS ist.

9. Verfahren zum Nachweisen und/oder zur Überwachung der Entwicklung einer disseminierten intravasalen Koagulopathie (DIC) ausgehend von einer biologischen Probe, umfassend die folgenden Schritte:

   a. Messen der Neutrophilenfluoreszenz $R_{0neutfl}$ mit einem DNA-interkalierenden Fluorochrom
   b. Vergleichen des gemessenen Neutrophilenfluoreszenz-Werts ROneutfl mit einem Referenzwert RNeutfl
   c. Messen der Konzentration $R_{0CD105}$ von Membranmikropartikeln, die den Cluster der Differenzierung 105 (CD105) umfassen,
   d. Vergleichen des gemessenen Werts $R_{0CD105}$ mit einem Referenzwert $R_{CD105}$,
   e. Messen der Blutplättchenkonzentration RoPlt
   f. Vergleichen des gemessenen Werts RoPlt mit einem Referenzwert RPlt
   g. Messen des Prothrombin-Gehalts RoTP
   h. Vergleichen des gemessenen Werts RoTP mit einem Referenzwert RTP
   i. Zuweisen eines Neutrophilenfluoreszenz-Werts Vneutfl gleich 1, wenn ROneutfl größer als der Wert $R_{neutfl}$ ist, oder eines Werts Vneutfl gleich 0, wenn ROneutfl kleiner als der Wert $R_{neutfl}$ ist
   j. Zuweisen eines Werts $V_{CD105}$ gleich 1, wenn $R_{0CD105}$ größer als der Wert $R_{CD105}$ ist, oder eines Werts CD105 gleich 0, wenn $R_{0CD105}$ kleiner als der Wert $R_{CD105}$ ist
   k. Zuweisen eines Werts $V_{Plq}$ gleich 1, wenn RoPlt kleiner als der Wert RPlt ist, oder eines Werts $V_{Plq}$ gleich 0, wenn RoPlt größer als der Wert RPlt ist
   l. Zuweisen eines Werts $V_{TP}$ gleich 1, wenn RoTP kleiner als der Wert RTP ist, oder eines Werts $V_{TP}$ gleich 0, wenn RoTP größer als der Wert RTP ist
   m. Berechnen des S1-Scores nach folgender Formel:

$$S_1 = \varepsilon + V_{neutfl} \times \alpha + V_{CD105} \times \beta + V_{Plq} \times \Omega + V_{TP} \times \theta$$

   worin $\varepsilon$ zwischen -0,708 und 2,280 beträgt, $\alpha$ zwischen 0,152 und 1,928 beträgt, $\beta$ zwischen 0,154 und 1,549 beträgt, $\Omega$ zwischen -0,016 und -0,005 beträgt, und $\theta$ zwischen -0,036 und 0,001 beträgt,
   oder

$$S1 = 0,786 + 1,040 \times V_{neutfl} + 0,851 \times V_{CD105} + (-0,010 \times V_{Plq}) + (-0,017 \times V_{TP})$$

10. Verfahren zum Nachweisen und/oder zur Überwachung der Entwicklung einer disseminierten intravasalen Koagulopathie (DIC) ausgehend von einer biologischen Probe, umfassend die folgenden Schritte:

   a. Messen der Neutrophilenfluoreszenz $R_{0neutfl}$ mit einem DNA-interkalierenden Fluorochrom

b. Vergleichen des gemessenen Werts der Neutrophilenfluoreszenz $R_{0neutfl}$ mit einem Referenzwert RNeutfl

c. Messen der Blutplättchenkonzentration RoPlt

d. Vergleichen des gemessenen Werts RoPlt mit einem Referenzwert RPlt

e. Messen des Prothrombin-Gehalts RoTP

f. Vergleichen des gemessenen Werts RoTP mit einem Referenzwert RTP

g. Zuweisen eines Neutrophilenfluoreszenz-Werts Vneutfl gleich 1, wenn ROneutfl größer als der Wert $R_{neutfl}$ ist, oder eines Werts Vneutfl gleich 0, wenn ROneutfl kleiner als der Wert $R_{neutsfl}$ ist

h. Zuweisen eines Werts $V_{Plq}$ gleich 1, wenn RoPlt kleiner als der Wert RPlt ist, oder eines Werts $V_{Plq}$ gleich 0, wenn RoPlt größer als der Wert RPlt ist

i. Zuweisen eines Werts $V_{TP}$ gleich 1, wenn RoTP kleiner als der Wert RTP ist, oder eines Werts $V_{TP}$ gleich 0, wenn RoTP größer als der Wert RTP ist

j. Berechnen des S2-Scores nach folgender Formel:

$$\text{S2} = \varepsilon + \text{Vneutfl} \times \alpha + V_{\text{Plättchen}} \times \Omega + V_{TP} \times \theta$$

worin $\varepsilon$ zwischen 0,381 und 3,070 beträgt, $\alpha$ zwischen 0,209 und 1,910 beträgt, $\Omega$ zwischen -0,015 und -0,005 beträgt, $\theta$ zwischen -0,040 und -0,004 beträgt.

oder

$$S2 = 1{,}726 + 1{,}060 * V_{neutfl} + (-0{,}010 * V_{Plq}) + (-0{,}022 * V_{TP})$$

11. Verfahren zum Nachweisen und/oder zur Überwachung der Entwicklung einer disseminierten intravasalen Koagulopathie (DIC) ausgehend von einer biologischen Probe, umfassend die folgenden Schritte:

a. Messen der Neutrophilenfluoreszenz $R_{0neutfl}$ mit einem DNA-interkalierenden Fluorochrom

b. Vergleichen des gemessenen Werts der Neutrophilenfluoreszenz ROneutfl mit einem Referenzwert RNeutfl

c. Messen der Konzentration $R_{0CD105}$ von Membranmikropartikeln, die den Cluster der Differenzierung 105 (CD105) umfassen,

d. Vergleichen des gemessenen Werts $R_{0CD105}$ mit einem Referenzwert $R_{CD105}$,

e. Messen der Blutplättchenkonzentration RoPlt

f. Vergleichen des gemessenen Werts RoPlt mit einem Referenzwert RPlt

g. Messen des Prothrombin-Gehalts RoTP

h. Vergleichen des gemessenen Werts RoTP mit einem Referenzwert RTP

i. Zuweisen eines Neutrophilenfluoreszenz-Werts Vneutfl gleich 1, wenn ROneutfl größer als der Wert Rneutsfi ist, oder eines Werts Vneutfl gleich 0, wenn ROneutfl kleiner als der Wert $R_{neutfl}$ ist

j. Zuweisen eines Werts $V_{CD105}$ gleich 1, wenn $R_{0CD105}$ größer als der Wert $R_{CD105}$ ist, oder eines Werts CD105 gleich 0, wenn $R_{0CD105}$ kleiner als der Wert $R_{CD105}$ ist

k. Zuweisen eines Werts $V_{Plq}$ gleich 1, wenn RoPlt größer als der Wert RPlt ist, oder eines Werts $V_{Plq}$ gleich 0, wenn RoPlt kleiner als der Wert RPlt ist

l. Zuweisen eines Werts $V_{TP}$ gleich 1, wenn RoTP größer als der Wert RTP ist, oder eines Werts $V_{TP}$ gleich 0, wenn RoTP kleiner als der Wert RTP ist

n. Berechnung des S1- und/oder S2-Scores nach folgender Formel:

$$S_1 = \varepsilon + V_{neutfl} \times \alpha + V_{CD105} \times \beta + V_{Plq} \times \Omega + V_{TP} \times \theta$$

worin $\varepsilon$ zwischen -0,708 und 2,280 beträgt, $\alpha$ zwischen 0,152 und 1,928 beträgt, $\beta$ zwischen 0,154 und 1,549 beträgt, $\Omega$ zwischen -0,016 und -0,005 beträgt, und $\theta$ zwischen -0,036 und 0,001 beträgt,

$$S_2 = \varepsilon + V_{neutfl} \times \alpha + V_{Plq} \times \Omega + V_{TP} \times \theta$$

worin $\varepsilon$ zwischen 0,381 und 3,070 beträgt, $\alpha$ zwischen 0,209 und 1,910 beträgt, $\Omega$ zwischen -0,015 und -0,005 beträgt, $\theta$ zwischen -0,040 und -0,004 beträgt.

**Claims**

1. An in-vitro process for predicting and/or detecting disseminated intravascular coagulation (DIC) from a first biological sample comprising a measurement of neutrophil fluorescence with a DNA intercalating fluorochrome.

2. The detection process as claimed in claim 1, further comprising a step of comparing the measured value with a reference value.

3. The detection process as claimed in claim 1 or 2, wherein the biological sample is selected from the group comprising a blood sample.

4. The process as claimed in any one of claims 1 to 3, also comprising a step of measurement, from a second biological sample, of the concentration $Rm_{CD105}$ of membrane microparticles bearing the cluster of differentiation 105 (CD105).

5. The process as claimed in any one of claims 1 to 4, wherein said first and second biological samples are the same sample.

6. The process as claimed in any one of claims 1 to 5, wherein said first and second biological samples originate from a patient with septic shock.

7. The process as claimed in any one of claims 1 to 6, wherein the neutrophil fluorescence reference value is greater than 57 AU.

8. The process as claimed in any one of claims 4 to 7, wherein the $Rm_{CD105}$ reference value is greater than 0.65 nM eq PS.

9. A process for detecting and/or monitoring the development of disseminated intravascular coagulation (DIC) from a biological sample, comprising the following steps:

   a. measuring neutrophil fluorescence ROneutfl with a DNA intercalating fluorochrome
   b. comparing the measured neutrophil fluorescence value ROneutfl with a reference value Rneutfl
   c. measuring the concentration $R_{0CD105}$ of membrane microparticles bearing the cluster of differentiation 105 (CD105)
   d. comparing the measured value $R_{0CD105}$ with a reference value $R_{CD105}$
   e. measuring the platelet concentration RoPlt
   f. comparing the measured value RoPlt with a reference value RPlt
   g. measuring the prothrombin content RoTP
   h. comparing the measured value RoTP with a reference value RTP
   i. assigning a neutrophil fluorescence value Vneutfl equal to 1 if ROneutfl is greater than the value $R_{neutfl}$ or a value Vneutfl equal to 0 if ROneutfl is less than the value $R_{neutfl}$
   j. assigning a value $V_{CD105}$ equal to 1 if $R_{0CD105}$ is greater than the value $R_{CD105}$ or a CD105 value equal to 0 if $R_{0CD105}$ is less than the value $R_{CD105}$
   k. assigning a value $V_{Plt}$ equal to 1 if RoPlt is less than the value RPlt or a value $V_{Plt}$ equal to 0 if RoPlt is greater than the value RPlt
   l. assigning a value $V_{TP}$ equal to 1 if RoTP is less than the value RTP or a value $V_{TP}$ equal to 0 if RoTP is greater than the value RTP
   m. calculating the score S1 according to the following formula:

$$S_1 = \varepsilon + V_{neutfl} \times \alpha + V_{CD105} \times \beta + V_{Plt} \times \Omega + V_{TP} \times \theta$$

wherein $\varepsilon$ is from -0.708 to 2.280, $\alpha$ is from 0.152 to 1.928, $\beta$ is from 0.154 to 1.549, $\Omega$ is from -0.016 to -0.005, and $\theta$ is from -0.036 to 0.001,
or

$$S1 = 0.786 + 1.040 \times V_{neutfl} + 0.851 \times V_{CD105} + (-0.010 \times V_{Plt}) + (-0.017 \times V_{TP}).$$

10. A process for detecting and/or monitoring the development of disseminated intravascular coagulation (DIC) from a biological sample, comprising the following steps:

   a. measuring neutrophil fluorescence ROneutfl with a DNA intercalating fluorochrome
   b. comparing the measured neutrophil fluorescence value ROneutfl with a reference value Rneutfl
   c. measuring the platelet concentration RoPlt
   d. comparing the measured value RoPlt with a reference value RPlt
   e. measuring the prothrombin level RoTP
   f. comparing the measured value RoTP with a reference value RTP
   g. assigning a neutrophil fluorescence value Vneutfl equal to 1 if ROneutfl is greater than the value $R_{neutfl}$ or a value Vneutfl equal to 0 if ROneutfl is less than the value $R_{neutsfl}$
   h. assigning a value $V_{Plt}$ equal to 1 if RoPlt is less than the value RPlt or a value $V_{Plt}$ equal to 0 if RoPlt is greater than the value RPlt
   i. assigning a value $V_{TP}$ equal to 1 if RoTP is less than the value RTP or a value $V_{TP}$ equal to 0 if RoTP is greater than the value RTP
   j. calculating the score S2 according to the following formula:

$$S2 = \varepsilon + V_{neutfl} \times \alpha + V_{platelets} \times \Omega + V_{TP} \times \theta$$

   wherein $\varepsilon$ is from 0.381 to 3.070, $\alpha$ is from 0.209 to 1.910, $\Omega$ is from -0.015 to -0.005, $\theta$ is from -0.040 to -0.004 or

$$S2 = 1.726 + 1.060 * V_{neutfl} + (-0{,}010 * V_{Plt}) + (-0.022 * V_{TP}).$$

11. A process for detecting and/or monitoring the development of disseminated intravascular coagulation (DIC) from a biological sample, comprising the following steps:

   a. measuring neutrophil fluorescence $R_{0neutfl}$ with a DNA intercalating fluorochrome.
   b. comparing the measured neutrophil fluorescence value ROneutfl with a reference value Rneutfl
   c. measuring the concentration $R_{0CD105}$ of membrane microparticles bearing the cluster of differentiation 105 (CD105)
   d. comparing the measured value $R_{0CD105}$ with a reference value $R_{CD105}$
   e. measuring the platelet concentration RoPlt
   f. comparing the measured value RoPlt with a reference value RPlt
   g. measuring the prothrombin level RoTP
   h. comparing the measured value RoTP with a reference value RTP
   i. assigning a neutrophil fluorescence value Vneutfl equal to 1 if ROneutfl is greater than the value $R_{neutsfl}$ or a value Vneutfl equal to 0 if ROneutfl is less than the value $R_{neutfl}$
   j. assigning a value $V_{CD105}$ equal to 1 if $R_{0CD105}$ is greater than the value $R_{CD105}$ or a value VCD105 equal to 0 if $R_{0CD105}$ is less than the value $R_{CD105}$
   k. assigning a value $V_{Plt}$ equal to 1 if RoPlt is greater than the value RPlt or a value $V_{Plt}$ equal to 0 if RoPlt is less than the value RPlt
   l. assigning a value $V_{TP}$ equal to 1 if RoTP is greater than the value RTP or a value $V_{TP}$ equal to 0 if RoTP is less than the value RTP
   n. calculating the score S1 and/or S2 according to the following formula:

$$S_1 = \varepsilon + V_{neutfl} \times \alpha + V_{CD105} \times \beta + V_{Plt} \times \Omega + V_{TP} \times \theta$$

   wherein $\varepsilon$ is from -0.708 to 2.280, $\alpha$ is from 0.152 to 1.928, $\beta$ is from 0.154 to 1.549, $\Omega$ is from -0.016 to -0.005, and $\theta$ is from -0.036 to 0.001,

$$S_2 = \varepsilon + V_{neutfl} \times \alpha + V_{Plt} \times \Omega + V_{TP} \times \theta$$

wherein $\varepsilon$ is from 0.381 to 3.070, $\alpha$ is from 0.209 to 1.910, $\Omega$ is from -0.015 to -0.005, $\theta$ is from -0.040 to -0.004.

Figure 1

Figure 2

**Figure 3**

Figure 4

Figure 5

**Figure 6A**

I.

II.

**Figure 6B**

**Figure 6C**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- US 20130101996 A

### Littérature non-brevet citée dans la description

- **KESSENBROCK K et al.** *Engelmann et Massberg,* 2015
- **STIEL et al.** Neutrophil Fluorescence: A New Indicator of Cell Activation During Septic Shock-Induced Disseminated Intravascular Coagulation. *Crit Care Med.,* 2016, vol. 44 (11), e1132-e1136
- **HUGEL B ; ZOBAIRI F ; FREYSSINET JM.** Measuring circulating cell-derived microparticles. *J Thromb Haemost.,* Octobre 2004, vol. 2 (10), 1846-7
- **AUPEIX K et al.** The significance of shed membrane particles during programmed cell death in vitro, and in vivo, in HIV-1 infection. *J Clin Invest,* 1997, vol. 99 (7), 1546-54
- **MCNAIR et al.** *JECT,* 2015, vol. 47, 113-118
- **DELABRANCHE et al.** *CCMED,* 2016
- **STIEL et al.** Neutrophil Fluorescence: A New Indicator of Cell Activation During Septic Shock-Induced Disseminated Intravascular Coagulation. *Crit Care Med.,* Novembre 2016, vol. 44 (11), e1132-e1136
- **R CORE TEAM.** R: A language and environment for statistical computing. *R Foundation for Statistical Computing,* 2012, http://www.Rproject.org
- **FOURRIER F.** Severe sepsis, coagulation, and fibrinolysis: dead end or one way?. *Crit Care Med,* 2012, vol. 40 (9), 2704-2708
- **GANDO S ; IBA T ; EGUCHI Y et al.** A multicenter, prospective validation of disseminated intravascular coagulation diagnostic criteria for critically ill patients: comparing current criteria. *Crit Care Med,* 2006, vol. 34 (3), 625-631
- **TAYLOR FB, JR. ; TOH CH ; HOOTS WK et al.** Towards définition, clinical and laboratory criteria, and a scoring system for disseminated intravascular coagulation. *Thromb Haemost,* 2001, vol. 86 (5), 1327-1330
- **DELABRANCHE X ; BOISRAME-HELMS J ; ASFAR P et al.** Microparticles are new biomarkers of septic shock-induced disseminated intravascular coagulopathy. *Intensive Care Med,* 2013, vol. 39 (10), 1695-1703

- **CHUNG S ; KIM JE ; PARK S et al.** Neutrophil and monocyte activation markers have prognostic impact in disseminated intravascular coagulation: in vitro effect of thrombin on monocyte CD163 shedding. *Thromb Res,* 2011, vol. 127 (5), 450-456
- **ENGELMANN B ; MASSBERG S.** Thrombosis as an intravascular effector of innate immunity. *Nature reviews Immunology,* 2013, vol. 13 (1), 34-45
- **ZONNEVELD R ; MOLEMA G ; PLOTZ FB.** Analyzing Neutrophil Morphology, Mechanics, and Motility in Sepsis: Options and Challenges for Novel Bedside Technologies. *Crit Care Med,* 2015
- **MATSUMOTO H.** The technology of reagents in the automated hematology analyzers Sysmex XE-2100™ - Red fluorescence technology. *Sysmex J Int,* 1999, vol. 9, 179-185
- **PARK SH ; PARK CJ ; LEE BR et al.** Sepsis affects most routine and cell population data (CPD) obtained using the Sysmex XN-2000 blood cell analyzer: neutrophil-related CPD NE-SFL and NE-WY provide useful information for detecting sepsis. *International journal of laboratory hematology,* 2015, vol. 37 (2), 190-198
- **ANGUS DC ; VAN DER POLL T.** Severe sepsis and septic shock. *N Engl J Med,* 2013, vol. 369, 840-851
- **VAN DER POLL T ; HERWALD H.** The coagulation system and its function in early immune defense. *Thromb Haemost,* 2014, vol. 112, 640-8
- **ENGELMANN B ; MASSBERG S.** Thrombosis as an intravascular effector of innate immunity. *Nat Rev Immunol.,* 2013, vol. 13, 34-45
- **WADA H ; MATSUMOTO T ; YAMASHITA Y.** Diagnosis and treatment of disseminated intravascular coagulation (DIC) according to four DIC guidelines. *J Intensive Care,* 2014, vol. 2, 15
- **GANDO S ; WADA H ; THACHIL J ; SCIENTIFIC.** Standardization Committee on DIC of the International Society on Thrombosis and Haemostasis. Differentiating disseminated intravascular coagulation (DIC) with the fibrinolytic phenotype from coagulopathy of trauma and acute coagulopathy of trauma-shock (COT/ACOTS). *J Thromb Haemost.,* 2013, vol. 11, 826-35

- **GANDO S ; OTOMO Y.** Local hemostasis, immuno-thrombosis, and systemic disseminated intravascular coagulation in trauma and traumatic shock. *Crit Care,* 2015, vol. 19, 72
- **FOURRIER F.** Severe sepsis, coagulation, and fibrinolysis: dead end or one way?. *Crit Care Med.,* 2012, vol. 40, 2704-8
- **LEVI M.** The coagulant response in sepsis and inflammation. *Hamostaseologie,* 2010, vol. 30, 10-2, 4-6
- **LEVI M ; VAN DER POLL T.** Endothelial injury in sepsis. *Intensive Care Med.,* 2013, vol. 39, 1839-42
- **MARSHALL JC.** Why have clinical trials in sepsis failed?. *Trends Mol Med.,* 2014, vol. 20, 195-203
- **MEZIANI F ; DELABRANCHE X ; ASFAR P et al.** Bench-to-bedside review: circulating microparticles--a new player in sepsis?. *Crit Care,* 2010, vol. 14, 236
- **DELABRANCHE X ; BOISRAME-HELMS J ; AS-FAR P et al.** Microparticles are new biomarkers of septic shock-induced disseminated intravascular coagulopathy. *Intensive Care Med.,* 2013, vol. 39, 1695-703
- **LEVY MM ; FINK MP ; MARSHALL JC et al.** Intensive Care Med. 2003. *SCCM/ESICM/ACCP/ATS/SIS International Sepsis Definitions Conference,* 2001, vol. 29, 530-8
- **TAYLOR FB, JR. ; TOH CH ; HOOTS WK et al.** Towards définition, clinical and laboratory criteria, and a scoring system for disseminated intravascular coagulation. *Thromb Haemost.,* 2001, vol. 86, 1327-30
- **GANDO S ; IBA T ; EGUCHI Y et al.** A multicenter, prospective validation of disseminated intravascular coagulation diagnostic criteria for critically ill patients: comparing current criteria. *Crit Care Med.,* 2006, vol. 34, 625-31
- **HUGEL B ; ZOBAIRI F ; FREYSSINET JM.** Measuring circulating cell-derived microparticles. *J Thromb Haemost,* 2004, vol. 2, 1846-7
- **SPERO JA ; LEWIS JH ; HASIBA U.** Disseminated intravascular coagulation. Findings in 346 patients. *Thromb Haemost,* 1980, vol. 43, 28-33
- **DELLINGER RP ; LEVY MM ; RHODES A et al.** Surviving sepsis campaign: international guidelines for management of severe sepsis and septic shock: 2012. *Crit Care Med.,* 2013, vol. 41, 580-637
- **TAGAMI T ; MATSUI H ; HORIGUCHI H et al.** Anti-thrombin and mortality in severe pneumonia patients with sepsis-associated disseminated intravascular coagulation: an observational nationwide study. *J Thromb Haemost.,* 2014, vol. 12, 1470-9
- **GANDO S ; SAITOH D ; OGURA H et al.** A multicenter, prospective validation study of the Japanese Association for Acute Medicine disseminated intravascular coagulation scoring system in patients with severe sepsis. *Crit Care,* 2013, vol. 17, R111
- **DE STOPPELAAR SF ; VAN 'T VEER C ; VAN DER POLL T.** The role of platelets in sepsis. *Thromb Haemost,* 2014, vol. 112, 666-77
- **HAMZEH-COGNASSE H ; DAMIEN P ; CHABERT A et al.** Platelets and infections - complex interactions with bacteria. *Front Immunol.,* 2015, vol. 6, 82
- **AUPEIX K ; HUGEL B ; MARTIN T et al.** The significance of shed membrane particles during programmed cell death in vitro, and in vivo, in HIV-1 infection. *J Clin Invest.,* 1997, vol. 99, 1546-54
- **LACROIX R ; ROBERT S ; PONCELET P et al.** Overcoming limitations of microparticle measurement by flow cytometry. *Semin Thromb Hemost.,* 2010, vol. 36, 807-18
- **ROBERT S ; LACROIX R ; PONCELET P et al.** High-sensitivity flow cytometry provides access to standardized measurement of small-size microparticles--brief report. *Arterioscler Thromb Vasc Biol.,* 2012, vol. 32, 1054-8
- **LACROIX R ; JUDICONE C ; MOOBERRY M ; BOUCEKINE M ; KEY NS ; DIGNAT-GEORGE F.** The ISSCW. Standardization of pre-analytical variables in plasma microparticle determination: results of the International Society on Thrombosis and Haemostasis SSC Collaborative workshop. *J Thromb Haemost.,* 2013, vol. 11, 1190-3
- **BRENNER T ; SCHMIDT K ; DELANG M et al.** Viscoelastic and aggregometric point-of-care testing in patients with septic shock - cross-links between inflammation and haemostasis. *Acta Anaesthesiol Scand,* 2012, vol. 56, 1277-90
- **PANIGADA M ; ZACCHETTI L ; L'ACQUA C et al.** Assessment of fibrinolysis in sepsis patients with urokinase modified thromboelastography. *PLoS One,* 2015, vol. 10, e0136463
- **HAASE N ; OSTROWSKI SR ; WETTERSLEV J et al.** Thromboelastography in patients with severe sepsis: a prospective cohort study. *Intensive Care Med,* 2015, vol. 41, 77-85